# EUROPÄISCHE PATENTANMELDUNG

(11) **EP 2 540 170 A1**
(43) Veröffentlichungstag der Anmeldung: **02.01.2013**
(21) Anmeldenummer: 11171953.0
(22) Anmeldetag: 29.06.2011
(51) Int. Cl.: A23L 1/30, A61K 8/99, A61K 35/68, A61Q 1/00

(54) **Dermatologisch wirksamer Hefeextrakt**

(71) Anmelder: Evonik Degussa GmbH, 45128 Essen (DE)
(72) Erfinder: Schiemann, Yvonne, 45659 Essen (DE); Farwick, Mike, 45138 Essen (DE); Haas, Thomas, 48161 Münster (DE); Wessel, Mirja, 44799 Bochum (DE)

(57) **Zusammenfassung**

Die Erfindung betrifft ein Verfahren zum Herstellen eines dermatologisch wirksamen Hefeextraktes, umfassend folgende Schritte Bereitstellen einer Vorkultur der Hefezellen" wenigstens fünfzehnminütiges Kultivieren der Zellen einem pH-Wert von 1,8 - 4, Ernten der Zellen und Lysieren der Zellen, sowie einen damit hergestellten Hefeextrakt und Produkte umfassend einen derartigen Hefeextrakt.

## Beschreibung

Die Erfindung betrifft ein Verfahren zum Herstellen eines dermatologisch wirksamen Hefeextraktes, umfassend folgende Schritte: Bereitstellen einer Vorkultur der Hefezellen, wenigstens fünfzehnminütiges Kultivieren der Zellen bei einem pH-Wert von 1,8 - 4, Ernten der Zellen und Lysieren der Zellen, sowie einen damit hergestellten Hefeextrakt und Produkte umfassend einen derartigen Hefeextrakt.

Die Haut ist das größte Organ des Menschen. Unter ihren vielen Funktionen, beispielsweise zur Wärmeregulation und als Sinnesorgan, ist die Barrierefunktion, die das Austrocknen der Haut - und damit letztlich des gesamten Organismus - verhindert, die wohl wichtigste. Gleichzeitig wirkt die Haut als Schutzeinrichtung gegen das Eindringen und die Aufnahme von außen kommenden Stoffen. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut. Die Epidermis ist ein stratifiziertes Gewebe, in dem die äußere Schicht, die Hornschicht (Stratum corneum), den für die Barrierefunktion bedeutenden Teil darstellt.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Oftmals wird mit der erschlafften Haut auch eine Begleiterscheinung der Übergewichtigkeit und/oder der damit häufig einhergehenden sogenannten Cellulite verbunden. Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhaut-fettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Vor diesem Hintergrund besteht zunehmend Bedarf an Agenzien, deren Anwendung auf die Haut eine Linderung oder zumindestens eine Verlangsamung der beschriebenen nachteiligen Effekte bewirkt, d.h. insbesondere eine hautstraffende und -stärkende Wirkung aufweist. Besonderer Bedarf besteht vor dem Hintergrund der Unsicherheit der Verbraucher mit Hinblick auf gentechnische Methoden an entsprechenden Agenzien, die nach strengen Maßstäben als rein biologisch gelten können, insbesondere solchen, die ohne Verwendung gentechnischer Methoden und/oder entsprechender Organismen erzeugt werden können.

WO2010087503 beschreibt die biotechnologische Verwendung von *Yarrowia* zur Herstellung von Succinat, lehrt jedoch nicht die Verwendung von Yarrowia-Extrakten für dermatologisch wirksame Agenzien.

Die der Erfindung zugrunde liegende Aufgabe besteht daran, neue dermatologisch wirksame Agenzien bereitzustellen, deren Anwendung auf die Haut den genannten Effekten entgegenwirkt, sowie Verfahren zur Herstellung derartiger Agenzien. Weiterhin besteht eine der vorliegenden Erfindung zugrunde liegende Aufgabe darin, ein nach strengen Kriterien rein biologisches dermatologisch wirksames Agens zu entwickeln, d.h. insbesondere eines, das ohne Verwendung gentechnischer Methoden oder gentechnisch veränderter Organismen hergestellt wird. Eine weitere der Erfindung zugrunde liegende Aufgabe der vorliegenden besteht darin, ein dermatologisch wirksames Agens zu entwickeln, das ausgehend von nachwachsenden Rohstoffen hergestellt werden kann.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Erfindungsgemäß wird die Aufgabe in einem ersten Aspekt gelöst durch ein Verfahren zum Herstellen eines dermatologisch wirksamen Hefeextraktes, umfassend folgende Schritte: a) Bereitstellen einer Vorkultur der Hefezellen, c) wenigstens fünfzehnminütiges Kultivieren der Zellen bei einem pH-Wert von 1,8 bis 4, d) Ernten der Zellen und e) Lysieren der Zellen.

In einer ersten Ausführungsform des ersten Aspektes umfasst das Verfahren weiter Schritt b) wenigstens einstündiges Kultivieren der Zellen bei einer Temperatur von 34 bis 39°C und einem pH-Wert > 5.

In einer zweiten Ausführungsform des ersten Aspektes, die auch eine Ausführungsform der ersten Ausführungsform darstellt, erfolgt Schritt e) unter Verwendung eines Lysemittels auf wässriger Basis.

In einer dritten Ausführungsform des ersten Aspektes, die auch eine Ausführungsform der ersten und zweiten Ausführungsform darstellt, wird zunächst Schritt b) und anschließend Schritt c) durchgeführt.

In einer vierten Ausführungsform des ersten Aspektes, die auch eine Ausführungsform der ersten bis dritten Ausführungsform darstellt, wird Schritt c) bei einer Temperatur von 34 bis 39°C durchgeführt.

In einer fünften Ausführungsform des ersten Aspektes, die auch eine Ausführungsform der ersten bis vierten Ausführungsform darstellt, dauert Schritt b) 3 bis 5 Stunden an.

In einer sechsten Ausführungsform des ersten Aspektes, die auch eine Ausführungsform der ersten bis fünften Ausführungsform darstellt, dauert Schritt c) 45 bis 75 Minutenan.

In einer siebten Ausführungsform des ersten Aspektes, die auch eine Ausführungsform der ersten bis sechsten Ausführungsform darstellt, werden die Schritte b) und c) bei einer Temperatur von 36 bis 38°C durchgeführt.

In einer achten Ausführungsform des ersten Aspektes, die auch eine Ausführungsform der ersten bis siebten Ausführungsform darstellt, wird Schritt c) bei einem pH-Wert von 1,9 bis 2,2 durchgeführt.

In einer neunten Ausführungsform des ersten Aspektes, die auch eine Ausführungsform der ersten bis achten Ausführungsform darstellt, handelt es sich bei der Hefezelle um eine Hefezelle aus der Gruppe von Gattungen, die *Yarrowia*, *Saccharomyces* und *Picchia* umfasst, und bevorzugt um *Yarrowia.*

In einer zehnten Ausführungsform des ersten Aspektes, die auch eine Ausführungsform der ersten bis neunten Ausführungsform darstellt, werden Schritt b) 3 bis 5 Stunden bei einer Temperatur von 36 bis 38°C und Schritt c) bei einem pH-Wert von 1,9 bis 2,2 und einer Temperatur von 36 bis 38°C und 45 bis 75 Minuten lang durchgeführt, und es handelt sich bei den verwendeten Hefezellen um Hefezellen der Gattung *Yarrowia.*

Erfindungsgemäß wird die Aufgabe in einem zweiten Aspekt gelöst durch einen Hefeextrakt, hergestellt nach dem ersten Aspekt oder einer der Ausführungsformen des ersten Aspektes.

Erfindungsgemäß wird die Aufgabe in einem dritten Aspekt gelöst durch ein dermatologisch wirksames Mittel, das ein Hefeextrakt aus Hefezellen der Gattung *Yarrowia* oder ein Hefeextrakt nach dem zweiten Aspekt der vorliegenden Erfindung umfasst.

In einer ersten Ausführungsform des dritten Aspektes der vorliegenden Erfindung wirkt das dermatologisch wirksame Mittel haut- und/oder gewebestraffend.

Erfindungsgemäß wird die Aufgabe in einem vierten Aspekt gelöst durch ein Nahrungs-ergänzungsmittel, das ein Hefeextrakt nach dem zweiten Aspekt umfasst.

In einer zweiten Ausführungsform des dritten Aspektes umfasst das dermatologisch wirksame Mittel nach dem dritten Aspekt oder einer seiner Ausführungsformen oder ein Nahrungs-ergänzungsmittel nach dem vierten Aspekt, wobei der Proteinanteil im Hefeextrakt 0,5 bis 50 mg/l beträgt.

Erfindungsgemäß wird die Aufgabe in einem fünften Aspekt gelöst durch ein Verfahren zur kosmetischen Behandlung von Haut und/oder Haar, umfassend eine topische Anwendung des Hefeextraktes nach dem zweiten Aspekt oder einem dermatologisch wirksamen Mittel nach dem dritten Aspekt oder einer der Ausführungsformen des dritten Aspektes.

Erfindungsgemäß wird die Aufgabe in einem sechsten Aspekt gelöst durch ein Hefeextrakt nach dem zweiten Aspekt der vorliegenden Erfindung zur Herstellung eines Medikamentes.

Erfindungsgemäß wird die Aufgabe in einem siebten Aspekt gelöst durch ein Hefeextrakt nach dem zweiten Aspekt der vorliegenden Erfindung zur Herstellung eines Medikamentes gegen Adipositas, Diabetes, Artherosklerose, entzündliche Krankheiten oder kardiovaskuläre Krankheiten oder zur Wundbehandlung.

Erfindungsgemäß wird die Aufgabe in einem achten Aspekt gelöst durch die Verwendung des Hefeextraktes nach dem zweiten Aspekt der vorliegenden Erfindung zur in vitro-Stimulierung der Proliferation von Keratinozyten, Fibroblasten und/oder Adipozyten.

Der vorliegenden Erfindung basiert auf der überraschenden Erkenntnis der Erfinder, dass die Verwendung alternativer Hefestämme und Bedingungen die Herstellung von Produkten erlaubt, die herkömmlichen Erzeugnissen gegenüber dermatologisch wirksamer sind. Insbesondere haben die Erfinder gefunden, dass Hefeextrakte auf der Basis von Stämmen der Gattung *Yarrowia* sich überraschend als besonders wirksam erweisen. Die Erfinder haben weiterhin gefunden, dass die Exposition von Hefezellen allgemein und Hefezellen von Stämmen der Gattung *Yarrowia* im Besonderen gegenüber einer Umgebung mit niedrigen pH-Werten eine geeignete Behandlung ist, um aus den genannten Zellen dermatologisch wirksame Agenzien herzustellen. Weiterhin haben die Erfinder der vorliegenden Erfindung gefunden, dass die dermatologische Wirksamkeit derartiger Agenzien überraschend durch eine Kombination der genannte Exposition der genannten Hefezellen gegenüber einer Umgebung mit niedrigen pH-Werten mit einer Exposition gegenüber Temperaturstress, bevorzugt in der Reihenfolge Temperaturstress und anschließendem pH-Stress, weiter verbessert werden kann. Schließlich haben die Erfinder der vorliegenden Erfindung gefunden, dass die Anwendung der genannten Hefeextrakte auf Hautmodelle, überraschenderweise den wnt-Signaltransduktionsweg und die extrazelluläre Matrix dieser Zellen reguliert und in für die Hautphysiologie vorteilhafter Weise beeinflusst.

Ohne an eine Theorie gebunden sein zu wollen, vermuten die Erfinder der vorliegenden Erfindung, dass die Hefezellen der Gattung *Yarrowia* deshalb einen dermatologisch wirksamen Extrakt abgeben, weil sie Zellmembranproteine aufweisen, die mit hydrophoben, fettreichen Substraten besonders gut wechselwirken, und/oder die Zellen einen oder mehrere bisher unidentifizierte Faktoren umfassen, die den Stoffwechsel von Hautzellen positiv beeinflussen.

Die vorliegende Erfindung umfasst sowohl Extrakte aus *Yarrowia*-Zellen, die auf jede denkliche Weise gestresst wurden, insbesondere durch Temperatur-, pH- und Oxidationsstress. Weiterhin umfasst die Erfindung durch extreme pH-Werte, bevorzugt niedrige pH-Werte, gestresste Hefezellen jeglicher Gattung. In einer bevorzugten Ausführungsform handelt es sich dabei um Zellen der Gattungen *Saccharomyces*, *Pichia* und *Yarrowia*, in einer bevorzugtereren Ausführungsform um Zellen der Gattung *Yarrowia,* insbesondere des Stammes *Yarrowia lipolytica.*

Es ist aus dem Stand der Technik bekannt, dass Hefezellen im sauren Milieu, d.h. bei pH-Werten unter 7 wachsen. Nichtsdestotrotz gibt es auch für Hefezellen Grenzen, und ab einem bestimmten pH-Wert zeigen sich Stressreaktionen, die mit dem sauren Medium zusammenhängen. In einer bevorzugten Ausführungsform bedeutet der Begriff "pH-Stress", wie hierin verwendet, dass die Hefezellen einem pH-Wert ausgesetzt sind, der unter dem für ihr Wachstum optimalen pH-Wert liegt. In einer noch bevorzugteren Ausführungsform liegt dieser pH Wert im Bereich von 1,8 bis 4. In einer noch stärker bevorzugten Ausführungsform liegt der pH-Wert im Bereich von 1,9 bis 2,2. Dem Fachmann sind Verfahren und Protokolle bekannt, nach denen der pH-Stress herbeigeführt werden kann, im einfachsten Fall die Senkung des Nährmediums durch Zugabe von Säure oder das Ernten der Zellen durch Zentrifugation, gefolgt von Resuspension in entsprechend saurerem Medium. Der pH-Stress, insbesondere in Form von Schritt c) nach dem ersten Aspekt der Erfindung, dauert zunehmend bevorzugt, wenigstens 20, 30, 45, 60, 75, 90, 120 oder 180 Minuten an oder dauert zunehmend bevorzugt 20 bis 180, 30 bis 90, 45 bis 75 oder 50 bis 70 Minuten an. Der pH-Wert beträgt in dieser Zeit zunehmend bevorzugt weniger als 5, 4,5, 4, 3,5, 3, 2,5 oder 2,2 oder liegt im Bereich von zunehmend bevorzugt 1,8 bis 5, 1,8 bis 4, 1,9 bis 3, 1,9 bis 2,5, 1,9 bis 2,2, 1,9 bis 2,2, 2 bis 2,8, 2 bis 2,2 und am bevorzugtesten bei 2.

In einer bevorzugten Ausführungsform wird unter dem Begriff "Lysieren" von Zellen, wie hierin verwendet, ein Verfahren verstanden, das geeignet ist, um eine Zelle in dem Sinne zu zerstören, dass ihre Membran durchlässig wird und Faktoren, die normal im Inneren der Zelle eingeschlossen sind, aus der Zelle austreten können. Dem Fachmann sind geeignete Verfahren zum Lysieren bekannt, beispielsweise durch die Verwendung von Ultraschall oder geeigneten, im Handel erhältlichen Geräten oder die Suspension in salzarmen Puffern oder destilliertem Wasser.

Bevorzugt erfolgt die Lyse der Hefezellen nach der pH-Stress-Behandlung unter Verwendung eines Lysemittels auf wässriger Basis. Unter diesem Begriff wird in einer bevorzugten Ausführungsform, wie hierin verwendet, verstanden, dass das Lysemittel überwiegend Wasser als Lösemittel umfasst. In einer weiteren Ausführungsform wie darunter verstanden, dass das Lösungsmittel ein anderes als ein Alkohol ist. In einer weiteren bevorzugten Ausführungsform ist das Lösungsmittel ein Alkohol, besonders bevorzugt Propandiol. Bei dem Hefeextrakt kann es sich in einer jeweils bevorzugten Ausführungsform um ein Totallysat oder um ein Lösungsmittelextrakt handeln.

Die Erfinder haben überraschend gefunden, dass die Kombination von Temperaturstress und pH-Stress sich besonders vorteilhaft auf die dermatologische Wirkung eines Hefeextraktes auswirkt. In einer besonders bevorzugten Ausführungsform wird unter dem Begriff "Temperaturstress", wie hierin verwendet, die Inkubation einer Hefezelle bei einer Temperatur verstanden, die über der für das Wachstum optimalen Temperatur liegt. In zunehmend bevorzugten Ausführungsformen ist diese Temperatur höher als 33, 34, 35, 36, 37, 38 oder 39 °C oder liegt im Bereich von 34 bis 42, 34 bis 39, 35 bis 38 oder 36 bis 38 °C. In einer besonders bevorzugten Ausführungsform liegt die Temperatur bei 37 °C. In einer besonders bevorzugten Ausführungsform ist der Behandlung der Hefezelle mit einem Medium unter pH-Stress eine Behandlung mit Temperaturstress direkt vorgeschaltet, d.h. die Hefezellen erhalten keine Gelegenheit, sich nach dem Temperaturstress zu erholen. In anderen Worten folgt auf Schritt b) nach dem ersten Aspekt der vorliegenden Erfindung unmittelbar Schritt c), beispielsweise durch Zugabe von Säure ins Medium in Schritt b). In einer besonders bevorzugten Ausführungsform wird die Stress auslösende Temperatur in Schritt c) beibehalten, d.h. die Hefezelle ist in Schritt c) einer Kombination von Temperatur- und pH-Stress ausgesetzt. In einer weiteren Ausführungsform ist die Reihenfolge der Schritte b) und c) beliebig. In einer weiteren bevorzugten Ausführungsform erfolgt Schritt c) während Schritt b) noch andauert, optional werden die Schritte b) und c) zusammen eingeleitet.

In einer bevorzugten Ausführungsform werden Schritt b) 3,5 bis 4,5 Stunden bei einer Temperatur von 37 °C und Schritt c) bei einem pH-Wert von 1,9 bis 2,1 und einer Temperatur von 36 bis 38°C und 45 bis 75 Minuten lang durchgeführt, und es handelt sich bei den verwendeten Hefezellen um Hefezellen der Gattung *Yarrowia.* In einer bevorzugten Ausführungsform werden Schritt b) 2 bis 6 Stunden bei einer Temperatur von 35 bis 39 °C und Schritt c) bei einem pH-Wert von 1,9 bis 2,1 und einer Temperatur von 36 bis 38°C und 45 bis 75 Minuten lang durchgeführt, und es handelt sich bei den verwendeten Hefezellen um Hefezellen der Gattung *Yarrowia lipolytica.*

Die Dauer des Temperaturstress-Schrittes b) muss so bemessen sein, dass die Zelle gezwungen ist, sich auf die veränderten, für sie unvorteilhaften Bedingungen einzustellen, insbesondere durch veränderte Expression von Proteinen, beispielsweise von Faktoren, die die Resistenz gegenüber der erhöhten Temperatur verbessern. In einer besonders bevorzugten Ausführungsform dauert Schritt b) nach dem ersten Aspekt der vorliegenden Erfindung zunehmend bevorzugt wenigstens 0,25, 0,5, 0,75, 1, 1,5, 2, 3 oder 4 Stunden an oder dauert 2 bis 10, 3 bis 6, 3 bis 5, 3,5 bis 4,5 Stunden an.

Die in dieser Anmeldung beschriebenen Hefeextrakte können zu dermatologisch wirksamen Pflegeformulierungen, auch synonym und austauschbar bezeichnet als dermatologisches Mittel, verarbeitet werden. In einer bevorzugten Ausführungsform wird unter dem Begriff "dermatologisches Mittel", wie hierin verwendet, ein Mittel zur nichtmedizinischen Behandlung der Haut verstanden. In einer bevorzugten Ausführungsform ist die Hefezelle eine Hefezelle aus der Gruppe umfassend die Gattungen *Yarrowia*, *Saccharomyces*, *Kluyveromyces*, *Torulaspora*, *Schizosaccharomyces*, *Debaromyces*, *Candida*, *Pichia*, *Aspergillus* and *Penicillium*, noch bervorzugter aus der Gruppe umfassend *Yarrowia, Saccharomyces* und *Pichia.* In einer besonders bevorzugten Ausführungsform ist die Hefezelle eine Hefezelle des Stammes *Yarrowia lipolytica.*

Die erfindungsgemäßen Pflegeformulierungen enthalten von 0,001 Massenprozent bis 20 Massenprozent, bevorzugt 0,01 Massenprozent bis 5 Massenprozent, besonders bevorzugt 0,05 Massenprozent bis 3, noch weiter bevorzugt 2 bis 3 Massenprozent Extrakt bezogen auf die Gesamtmasse der Pflegeformulierung.

Die erfindungsgemäßen Pflegeformulierungen können mindestens eine zusätzliche Komponente enthalten, die aus der Gruppe umfassend Emollients, Emulgatoren und Tenside, Verdicker/Viskositätsregler/Stabilisatoren, UV-Lichtschutzfilter, Antioxidantien und Vitamine, Hydrotrope (oder Polyole), Fest- und Füllstoffe, Filmbildner,Perlglanzadditive, Deodorant- und Antitranspirantwirkstoffe, Insektrepellentien, Selbstbräuner, Konservierungsstoffe, Konditioniermittel, Parfüme, Farbstoffe, Biogene Wirkstoffe, Pflegeadditive, Überfettungsmittel und Lösungsmittel ausgewählt ist.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat möglich. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyl-adipat, Di-n-butyl-sebacat, Di-(2-ethylhexyl)-adipat, Di-(2-hexyldecyl)-succinat, D-isotridecylacelaat. Geeignete Diolester sind z.B. Ethylenglycoldioleat, Ethylenglycol-di-isotridecanoat, Propylenglycol-di-(2-ethylhexanoat), Butandiol-di-isostearat, Butandiol-di-caprylat/caprat und Neopentylglycol-di-caprylat. Weitere Fettsäureester, die als Emollients eingesetzt werden können, sind z.B. C₁₂-₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Kamaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl-oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen C6- C22-Fettalkoholen, Ester von verzweigten C6-C13-Carbonsäuren mit linearen C6-C22-Fettalkoholen, Ester von linearen C6-C22-Fettsäuren mit verzweigten C8-C18-Alkoholen, insbesondere 2-Ethylhexanol oder Isononanol, Ester von verzweigten C6-C13-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C6-C22-Fettalkoholcarbonate, Guerbetcarbonate, Ester der Benzoesäure mit linearen und/oder verzweigten C6-C22-Alkoholen (z. B. Finsolv^{™} TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als Emulgatoren oder Tenside können nichtionische, anionische, kationische oder amphotere Tenside eingesetzt werden. Als nichtionogene Emulgatoren oder Tenside können Verbindungen aus mindestens einer der folgenden Gruppen eingesetzt werden: Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C_{12/18}-Fettsäuremono-und -diester von Anlagerungsprodukten von 1 bis 100 Mol Ethylenoxid an Glycerin, Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte, Anlagerungsprodukte von 2 bis 200 Mol Ethylenoxid an Ricinusöl und/oder gehärtetes Ricinusöl, Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose), Mono-, Di- und Trialkylphosphate sowie Mono-, Di- und/oder Tri-PEG-alkylphosphate und deren Salze, Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15, Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL^{®} EM 90 (Evonik Goldschmidt GmbH)), Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z.B. Glycerin oder Polyglycerin, Zitronensäureester wie z.B. Glyceryl Stearate Citrate, Glyceryl Oleate Citrate und Dilauryl Citrate.

Anionische Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium-oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder - bromid oder Behenyltrimethylammoniumchlorid, aber auch Dialkyldimethylammoniumhalogenide wie z.B. Distearyldimethylammoniumchlorid eingesetzt werden.

Weiterhin können Monoalklyamidoquats wie z.B. Palmitamidopropyl-trimethylammoniumchlorid oder entsprechende Dialkylamidoquats eingesetzt werden.

Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.

Typische Beispiele für milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen.

Weiterhin ist es möglich, amphotere Tenside wie z.B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Von den ampholytischen Tensiden können solche oberflächenaktiven Verbindungen eingesetzt werden, die außer einer C8/18-Alkyl- oder -Acylgruppe im Molekül mindestens eine freie Aminogruppe und mindestens eine -COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C12/18-Acylsarcosin.

Geeignete Verdicker sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z. B. Carbopole TM oder Synthalene TM ), Polyacrylamide, Polyvinylalkohol und Polyvinylpyrrolidon, Tenside wie beispielsweise ethoxylierte Fettsäure-glyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und /oder Metallsalze von Fettsäuren, wie z.B. Zinkstearat eingesetzt werden.

Als Viskositätsregler für wässrige Tensidsysteme können z.B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen: 3-Benzylidencampher und dessen Derivate, z.B. 3-(4-Methylbenzyliden)campher, 4-Aminobenzoesäurederivate, wie z.B. 4-(Dimethylamino)benzoesäure-2-ethylhexylester,4-(Dimethylamino)benzoesäure-2-ethylhexylester und 4-(Dimethylamino)-benzoesäureamylester, Ester der Zimtsäure, wie z.B. 4-Methoxyzimtsäure-2-ethylhexylester, 4-Methoxyzimt-säureisopentylester, 2-Cyan-3-phenyl-zimtsäure-2-ethylhexylester (Octocrylene), Ester der Salicylsäure, wie z.B. Salicylsäure-2-ethylhexylester, Salicylsäure-4-isopropylbenzylester, Salicylsäurehomomenthylester, Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon, Ester der Benzalmalonsäure, wie z.B. 4-Methoxybenzmalonsäuredi-2-ethylhexyester, Triazinderivate, wie z.B. 2,4,6-Trianilino-(p-carbo-2'-ethyl-1'-hexyloxy)-1,3,5-triazin und Octyltriazon, Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage: 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenon, wie z.B. 2-Hydroxy-4-methoxybenzophenon-5-sulfonsäure und ihre Salze, Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze. Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen. Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Als Antioxidantien und Vitamine können z.B. Superoxid-Dismutase, Tocopherol (Vitamin E), Tocopherolsorbat, Tocopherolacetat, andere Ester von Tocopherol, Dibutylhydroxytoluol und Ascorbinsäure (Vitamin C) und ihre Salze sowie deren Derivate (z.B. Magnesium Ascorbylphosphat, Natrium Ascorbylphosphat, Ascorbylsorbat), Ascorbyl Ester von Fettsäuren, butylierte Hydroxybenzoesäure und ihre Salze, Peroxide wie z.B. Wasserstoffperoxid, Perborate, Thioglycolate, Persulfatsalze, 6-Hydroxy-2,5,7,8-Tetramethylchroman-2-Carboxylsäure (TROLOX.RTM), Gallussäure und ihre Alkylester, Harnsäure und ihre Salze und Alkylester, Sorbinsäure und ihre Salze, Liponsäure, Ferulasäure, Amine (z.B. N,N-Diethylhydroxylamin, Amino-Guanidine), Sulfhydryl-Verbindungen (z.B. Glutathion), Dihydroxy-Fumarsäure und ihre Salze, Glycinpidolat, Argininpilolat, Nordihydroguaiaretissche Säure, Bioflavonoide, Curcumin, Lysin, L-Methionin, Prolin, Superoxid Dismutase, Silymarin, Tee Extract, Grapefruit Schalen/Kern Extrakt, Melanin, Rosmarin Extrakt, Thioctansäure, Resveratrol, Oxyresveratrol, etc. eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele umfassen: Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%, Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere. Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filternoch färbende Wirkung haben, beispielsweise Aerosile® (CAS-Nr. 7631-86-9).

Als Filmbildner zur z.B. Verbesserung der Wasserfestigkeit können beispielsweise eingesetzt werden: Polyurethane, Dimethicone, Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z.B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Ilit, Beidelit, Nontronit, Saponit, Ilectorit, Bentonit, Smectit, ferner beispielsweise Zinksalze der Ricinolsäure. keimhemmende Mittel sind ebenfalls geeignet, eingearbeitet zu werden. keimhemmende Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO^{®} Cosmo P813, Evonik Goldschmidt GmbH), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methlyparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethylisothiazolin, Ethylhexylglycerin oder Caprylyl Glycol eingesetzt werden.

Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid, Palmitamidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Polyphenole, Desoxyribonucleinsäure, Coenzym Q10, Retinol, AHA-Säuren, Aminosäuren, Hyaluronsäure, alpha-Hydroxysäuren, Isoflavone, Polyglutaminsäure, Creatin (und Creatinderivate), Guanidin (und Guanidinderivate), Pseudoceramide, essentielle Öle, Peptide, Proteinhydrolysate, Pflanzenextrakte, Bisabolol, Allantoin, Panthenol, Phytantriol, Idebenon, Lakritz Extrakt, Glycyrrhizidin und Idebenon, Skleroglucan, ß-Glucan, Santalbinsäure und Vitaminkomplexe zu verstehen. Beispiele für Pflanzenextrakte sind Kastanien Extrakt, Kamillen Extrakt, Rosmarin Extrakt, schwarze und rote Johannisbeer Extrakt, Birken Extrakt, Hagebutten Extrakt, Algen Extrakte, Grüner Tee Extrakt, Aloe Extrakt, Ginseng Extrakt, Ginko Extrakt, Grapefruit Extrakt, Calendula Extrakt, Kampher, Thymus Extrakt, Mangosteen Extrakt, Cystus Extrakt, Terminalia Arjuna Extrakt, Hafer Extrakt, Oregano Extrakt, Himbeer Extrakt, Erdbeer Extrakt, etc.

Zu den biogenen Wirkstoffen können auch die sogenannten Barriere Lipids gezählt werden, für welche beispielhaft Ceramide, Phytosphingosin und Derivate, Sphingosin und Derivate, Sphinganin und Derivate, Pseudoceramide, Phospholipide, Lysophospholipide, Cholesterin und Derivate, Cholesteryl Ester, freie Fettsäuren, Lanolin und Derivate, Squalan, Squalen und verwandte Substanzen genannt werden.

Zu den biogenen Wirkstoffen werden im Sinne der Erfindung auch anti-Akne wie z.B. Benzylperoxid, Phytosphingosin und Derrivate, Niacinamid Hydroxybenzoat, Nicotinaldehyd, Retinolsäure und Derrivate, Salicylsäure und Derivate, Citronellsäure etc. und anti-Cellulite wie z.B. Xanthin Verbindungen wie Coffein, Theophyllin, Theobromin und Aminophyllin, Carnitin, Carnosin, Salicyloyl Phytosphingosin, Phytosphingosine, Santalbinsäure etc. gezählt, ebenso wie Antischuppenmittel wie beispielsweise Salicylsäure und Derrivate, Zink Pyrithion, Selensulfid, Schwefel, Ciclopiroxolamin, Bifonazol, Climbazol, Octopirox und Actirox etc, ebenso wie Adstringetien wie z.B. Alkohol, Aluminium Derivate, Gallsäure, Pyridoxinsalicylat, Zinksalze wie z.B. Zinksulfat, -acetat, -chlorid, -lactat, Zirconiumchlorohydrate etc. Ebenso können Bleichmittel wie Kojisäure, Arbutin, Vitamin C und Derivate, Hydroquinon, Turmeric oil, Creatinin, Sphingolipide, Niacinamid, etc. zu den biogenen Wirkstoffen gezählt werden.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z.B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.

Es ist bevorzugt, dass die erfindungsgemäßen Pflegeformulierungen als zusätzliche Komponente Barrierelipide ausgewählt aus der Gruppe enthaltend Ceramide, Cholesterol, Fettsäuren.

Des Weiteren ist es bevorzugt, dass die erfindungsgemäßen Pflegeformulierungen als zusätzliche Komponenten Lipidmodulatoren wie beispielsweise Linolensäure, konjugierte Linolsäure, gamma Linolensäure,

Phytosphingosin, Salicyloyl-Phytosphingosin, kurz- und mittelkettige Ceramide (C1-C10), Uronsäure, Cholesterolsulfat, Phytosterole,Vitamin-D, Leukotriene, Farnesol, 15-Deoxyprostaglandin J2, 9-Hydroxyoktadekadien-Säure (9-HODE) enthalten, bevorzugt Lipidmodulatoren ausgewählt aus der Gruppe enthaltend Creatin, Niacinamid, Retinol, Arjunolic acid.

Erfindungsgemäße Pflegeformulierungen können Verwendung als Hautpflege-, Gesichtspflege-, Kopfpflege-, Körperpflege-, Intimpflege-, Fußpflege-, Haarpflege-, Nagelpflege-, Zahnpflege-, Lippenpflege- oder Mundpflegeprodukt finden. Beispiele für Haarpflegeprodukte sind Haarwaschmittel, Haarkuren, Haarspülungen, Haarfluid, Haargel, Haartonic, Haarwachs, Haarlack, Haarspray, Haarcreme, Haarmousse, Haarbalsam, Antischuppenshampoo. Beispiele für Körperpflegeprodukte sind Duschbad, Cremebad, Cremegel, Duschöl, Duschgel, Waschgel, Waschpeeling, Reinigungslotion, Gesichtsmaske, Gesichtswasser, Gesichtspeeling, Augencreme, Nachtcreme, Reinigungsmaske, Lotion pads, Reinigungstücher, Reinigungslotion, Reinigungsmilch, Reinigungsgel, After Shave Gel, After Shave Balsam, Sonnenmilch, After Sun Produkte, Selbstbräuner, Fusslotion, Fussspray, Körperlotion, Körpergel, Körperspray, Körpermilch, Körperpeeling, Körperöl, Körperbutter.

Beispiele für Lippenpflegeprodukte sind Lippenbalsam, Lippencreme, Lippenpflegestift.

Erfindungsgemäße Pflegeformulierungen können Verwendung in Form einer Emulsion wie Öl-in-Wasser- (O/W), Wasser-in-Öl- (W/O) oder Wasser-in Silikon- (W/S) Emulsionen, multiple Emulsionen wie W/O/W- und O/W/O-Emulsionen, auch als Hydrodispersionen oder Lipodispersionen bezeichnet, einer Suspension, einer Lösung, einer Creme, einer Salbe, einer Paste, eines Gels, eines Aerosols, eines Sprays, eines Reinigungsproduktes, eines Schmink- oder Sonnenschutzpräparates oder eines Gesichtswassers oder eines Stiftes, z. B. Fettstiftes oder wasserhaltigen Stiftes, finden.

Das erfindungsgemäß hergestellte Hefeextrakt kann auch als Nahrungsergänzungsmittel für Tiere und/oder Menschen Verwendung finden. Die Verabreichung eines derartigen Extraktes kann mit der Nahrung erfolgen.

Ein dermatologisch wirksames Mittel bzw. ein Nahrungsergänzungsmittel muss eine bestimmte Mindestmenge an Protein im Hefeextrakt enthalten. In einer bevorzugten Ausführungsform enthält der Hefeextrakt zunehmend bevorzugt wenigstens 0,5, 1, 2,5, 5, 10 oder 25 mg Protein/ml oder liegt im Bereich von 0,05 bis 100, 0,1 bis 80, 0,5 bis 50 oder 1 bis 25 mg Protein/ml.

Das erfindungsgemäß hergestellte Hefeextrakt kann bei kosmetischen Behandlungen von Haar und/oder Haut eingesetzt werden. In einer bevorzugten Ausführungsform ist unter dem Begriff "Haut und/oder Haar", wie hierin verwendet, jeder Teil des menschlichen oder tierischen Körpers zu verstehen, der eine äußerlichen Anwendung eines dermatologischen Mittels zugänglich ist. In einer bevorzugten Ausführungsform bedeutet der Begriff "kosmetische Behandlung", wie hierin verwendet, die Verbesserung nicht krankhafter Erscheinungen, die insbesondere lediglich den ästhetischen Eindruck verbessert. Mit einer derartigen Behandlung kann beispielsweise eine Hautstraffung, ein verbesserte Hautfeuchte, ein ebenmäßiger Hautton, oder eine Verbesserung der Hautatmung erreicht werden.

Die Erfinder haben überraschend gefunden, dass die Behandlung von Hautzellen mit dem erfindungsgemäß hergestellten Hefeextrakt zur Behandlung von Krankheiten geeignet ist.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.
**Fig. 1** zeigt das Wachstum von Hefezellen der Gattungen *Yarrowia*, *Pichia* und *Saccharomyces* über die Zeit bei pH 2.
**Fig. 2** zeigt zwei repräsentative Sets mit Chipdaten, wie sie unter Verwendung des beschriebenen Protokolls erhalten wurden. Die Daten dort wurden durch Analyse der Marker von humanen Fibroblasten erhalten, die mit Extrakt aus *Yarrowia*-Zellen behandelt wurden, die normal bei pH 5,4 kultiviert wurden **(****Fig. 2a****)** oder Stress bei pH 2 ausgesetzt wurden **(****Fig. 2b****).**

### Beispiel 1: Wachstum verschiedener Hefestämme bei Stress durch Exposition gegenüber Medium mit niedrigem pH

Um zu untersuchen, ob es möglich ist, Hefestämme durch Exposition gegenüber Medium mit niedrigen pH-Werten zu stressen, und zu untersuchen, welche Hefestämme ggf. bei niedrigen pH-Werten geeignete oder vorteilhafte Wachstumseigenschaften zeigen, wurde das Wachstum von je einem Vertreter dreier Gattungen, nämlich *Saccharomyces*, *Pichia* und *Yarrowia*, bei pH = 2 untersucht.

Eine Vorkultur des jeweiligen zu untersuchenden Hefestammes wird über Nacht in Standardmedium (pro Liter 33 g Glucose-Monohydrat, 0,88 g Magnesiumsulfat x 7 H₂O, 0,2 g Calciumchlorid x 2 H₂O, 4,83 Ammoniumchlorid, 0,06 g Natriumchlorid, 1 g Kaliumdihydrogenphosphat, 0,059 g Myo-Inositol, 20 g MOPS, 0,3 ml Spurenelementlösung (pro kg 100 g 8 M H₂SO₄, 50 g des Monohydrates der Zitronensäure, 48 g FeSO₄x7H₂O, 16,7 g ZnSO₄x7H₂O, 2,5 g CuSO₄x5H₂O, 1,88 g MnSO₄xH₂O, 2 g H₃BO₃, 2 g NaMoO₄x2H₂O, 0,5 g KI) und 0,3 ml Vitaminlösung (pro Liter 5 g Nikotinsäure, 5 g Calcium-D-Pantothensäure, 5 g Thiamin, 3,33 p-Aminobenzoat, 0,5 g Pyridoxin, 0,0167 Biotin) herangezogen, dessen pH mit Schwefelsäure auf 2 eingestellt wurde. Die optische Dichte wird kontinuierlich spektrophotometrisch verfolgt.

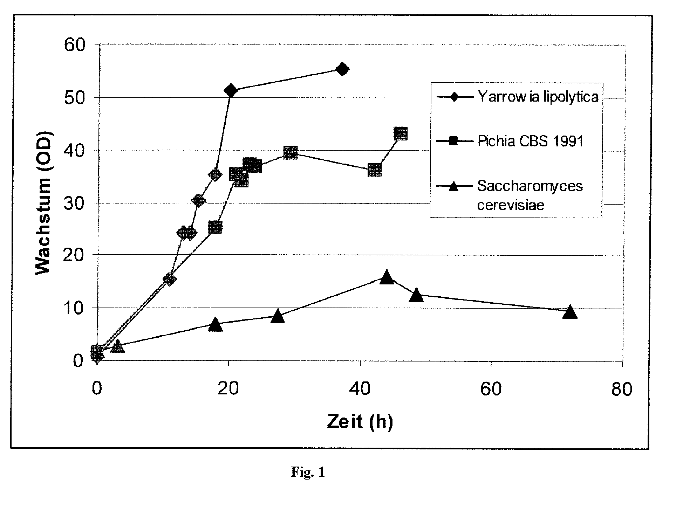

Die Ergebnisse des Versuchs sind in **Fig. 1** dargestellt. Es zeigt sich, dass alle verwendeten Stämme unter diesen Bedingungen ein detektierbares Wachstum zeigen, dass aber der Vertreter der Gattung *Yarrowia* bei pH = 2 unter gleichen Bedingungen gegenüber den Vergleichsgattungen ein schnelleres Wachstum zeigt und zudem eine höhere optische Dichte erreicht.

### Beispiel 2: Vergleichende Charakterisierung der Wirkung von Hefezellen, die entweder durch erhöhte Temperatur, Behandlung mit Wasserstoffperoxid oder Exposition gegenüber Medium mit niedrigem pH gestresst wurden, unter Verwendung von Chiparrays auf humane Fibroblasten

Es wurde untersucht, ob Stress durch Exposition gegenüber Medium mit niedrigem pH die gleiche Wirkung auf Hefezellen ausübt wie Stress durch erhöhte Temperatur oder Behandlung mit Wasserstoffperoxid und ob sich unterschiedlich gestresste Hefezellen bzw. ihre Extrakte unterscheiden. Als Modellorganismus wurde *Saccharomyces cerevisiae* gewählt.

### Herstellung des Hefeextraktes

Die Vorkultur wird angesetzt wie oben beschrieben und auf einen pH Wert von 5,4 eingestellt. Das Medium wird im Schüttelkolben ohne Schikanen mit Antischaumzugabe angeimpft. Die Temperatur beträgt 30°C und die Geschwindigkeit von 180 rpm (Amplitude: 2,5cm). Vorab wird die optische Dichte bestimmt, diese wird kontinuerlich gemessen, und bei einer OD > 35 wird die jeweilige Stresssituation hervorgerufen, d.h. durch Erhöhung der Temperatur auf 37 °C, Hinzugabe von Schwefelsäure bis zum erwünschten pH oder durch Zugabe von Wasserstoffperoxid.

Anschließend werden die Zellen durch Zentrifugation geerntet und eingefroren bzw. aufgeschlossen mittels wässrigen Aufschluss. Dazu wird der Extrakt suspendiert und mittels Hochdruckhomogenisator behandelt. Die Suspension wird abfiltriert bzw. zentrifugiert und die wässrige Lösung als Endprodukt entnommen

### Versuchsprinzip und -durchführung der Experimente mit Chiparrays:

Bei diesen Versuchen wird die Wirkung von Hefeextrakt auf das Verhalten von humanen dermalen Fibroblasten untersucht, die ein anerkanntes Modell für die menschliche Haut darstellen. Zu diesem Zweck werden die Zellen nach der Extraktbehandlung aufgeschlossen, ihre RNA isoliert, und die durch reverse Transkription erhaltene DNA wird mittels eines Chips auf die Regulation der Expression dermatologisch relevanter Marker untersucht. Dabei werden ausschließlich Marker verwendet, deren Herauf- bzw. Runterregulation eine positive Wirkung auf die Morphologie und Physiologie der Haut anzeigt, insbesondere Marker des wnt-Pathways und der ECM. Die Wnt-Gene kodieren für eine große Familie sezernierter Proteine. Beim Menschen wurden bisher 19 Wnt-Proteine identifiziert. Sowohl die verschiedenen Untergruppen der Wnts in einzelnen Spezies, als auch Wnts im Vergleich zwischen verschiedenen Spezies zeigen zum Teil sehr große Homologien. Beim Menschen beispielsweise zeigen die einzelnen Wnt- Proteine eine Übereinstimmung von 27 bis 83%. (Miller, 2001) Das zentrale Molekül der kanonischen Wnt-Signalkaskade ist das β-Catenin (βCat), dessen Stabilität durch die Wnt-Proteine reguliert wird. Untersuchungen an embryonaler und postnataler Haut haben gezeigt, dass Wnt-Proteine bei der Haarfollikelentwicklung eine essentielle Rolle spielen. Insbesondere die Proteine Wnt10a, Wnt10b und Wnt5a werden in frühen Stadien der Haarfollikelentwicklung spezifisch exprimiert. (Reddy et al., 2001).

In der Haut findet man die meisten bekannten Kommunikationsmechanismen wieder. Durch den Wntpathway behalten Basalzellen bevorzugt den Stammzellcharakter. Beim Altern von Zellen kommt es zu einer Anhäufung von zellulären und molekularen Schäden, die eine verringerte Regenerationsfähigkeit von Geweben und Organen bewirken. Diese verringerte Regenerationsfähigkeit wird teilweise durch die Veränderung des Selbsterneuerungsvermögens und des Differenzierungspotentials von gewebsspezifischen adulten Stammzellen aus dem Fettgewebe beeinflusst. Es ist bekannt, das der WNT Signalweg, ein bekannter Signalweg, der das Selbsterneuerungspotential und das Differenzierungspotential vieler Stammzellen reguliert und somit eine Wirkung auf das Wachstum der Stammzellen hat. Ebenfalls wird mit der Wnt-und β Catenin Expression die epidermale Proliferation, Differenzierung und Migration erhöht und somit die Wundheilung beschleunigt.

Die physikalischen Eigenschaften des Bindegewebes und die damit verbundene Hautstraffung werden unter anderem durch die extrazelluläre Matrix bestimmt, wodurch die Zellen in ihrer Differenzierung, Migration und Proliferation beeinflusst werden. Die Matrix wird von den in ihr liegenden Zellen (Fibroblasten, Myofibroblasten, Lipoblasten, Osteoblasten und Chondroblasten) gebildet. Die extrazelluläre Matrix ist aus Makromolekülen aufgebaut, welche aus Glykosaminoglykane und Faserproteine bestehen. Ein Zusammenspiel des Lymphsystem, der extrazellulären Matrix und eine exzessive Fett-Akkumulation sind bei der Hautstraffung involviert. Das gesamte Körperfett wird durch das Gleichgewicht zwischen De-novo-Differenzierung, Wachstum und Apoptose (Selbstauflösung) der Adipozyten bestimmt. Die Adipozytendifferenzierung ist ein komplexer Prozess, der mit der Geburt beginnt und das ganze Leben anhält.

Eine vollständige Liste der verwendeten Marker ist in **Tabelle 1** abgebildet.

**Tabelle 1: Liste der für die Chipexperimente berücksichtigten Marker:**

| Gen ID | Gen-Symbol | Beschreibung des Gens | Chromosom |
|---|---|---|---|
| 9 | NAT1 | N-acetyltransferase 1 (arylamine N-acetyltransferase) | 8p23.1-p21.3 |
| 10 | NAT2 | N-acetyltransferase 2 (arylamine N-acetyltransferase) | 8p22 |
| 30 | ACAA1 | acetyl-Coenzyme A acyltransferase 1 | 3p23-p22 |
| 33 | ACADL | acyl-Coenzyme A dehydrogenase, long chain | 2q34-q35 |
| 34 | ACADM | acyl-Coenzyme A dehydrogenase, C-4 to C-12 straight chain | 1p31 |
| 51 | ACOX1 | acyl-Coenzyme A oxidase 1, palmitoyl | 17q24-q25 |
| 90 | ACVR1 | activin A receptor, type I | 2q23-q24 |
| 91 | ACVR1B | activin A receptor, type IB | 12q13 |
| 92 | ACVR2A | activin A receptor, type IIA | 2q22.3 |
| 93 | ACVR2B | activin A receptor, type IIB | 3p22 |
| 94 | ACVRL1 | activin A receptor type II-like 1 | 12q11-q14 |
| 154 | ADRB2 | adrenergic, beta-2-, receptor, surface | Sq31-q32 |
| 176 | ACAN | aggrecan | 15q26.1 |
| 268 | AMH | anti-Mullerian hormone | 19p13.3 |
| 269 | AMHR2 | anti-Mullerian hormone receptor, type II | 12q13 |
| 324 | APC | adenomatous polyposis coli | 5q21-q22 |
| 325 | APCS | amyloid P component, serum | 1q21-q23 |
| 335 | APOA1 | apolipoprotein A-1 | 11q23-q24 |
| 336 | APOA2 | apolipoprotein A-II | 1q21-q23 |
| 345 | APOC3 | apolipoprotein C-III | 11q23.1-q23.2 |
| 364 | AQP7 | aquaporin 7 | 9p13 |
| 387 | RHOA | ras homolog gene family, member A | 3p21.3 |
| 595 | CCND1 | cyclin D 1 | 11q13 |
| 633 | BGN | biglycan | Xq28 |
| 650 | BMP2 | bone morphogenetic protein 2 | 20p12 |
| 652 | BMP4 | bone morphogenetic protein 4 | 14q22-q23 |
| 653 | BMP5 | bone morphogenetic protein 5 | 6p12.1 |
| 654 | BMP6 | bone morphogenetic protein 6 | 6p24-p23 |
| 655 | BMP7 | bone morphogenetic protein 7 | 20q13 |
| 656 | BMP8B | bone morphogenetic protein 8b | 1p35-p32 |
| 657 | BMPR1A | bone morphogenetic protein receptor, type IA | 10q22.3 |
| 658 | BMPR1B | bone morphogenetic protein receptor, type IB | 4q22-q24 |
| 659 | BMPR2 | bone morphogenetic protein receptor, type II (serine/threonine kinase) | 2q33-q34 |
| 815 | CAMK2A | calcium/calmodulin-dependent protein kinase II alpha | 5q33.1 |
| 816 | CAMK2B | calcium/calmodulin-dependent protein kinase II beta | 22q12 |
| 817 | CAMK2D | calcium/calmodulin-dependent protein kinase II delta | 4q26 |
| 818 | CAMK2G | calcium/calmodulin-dependent protein kinase II gamma | 10q22 |
| 894 | CCND2 | cyclin D2 | 12p13 |
| 896 | CCND3 | cyclin D3 | 6p21 |
| 948 | CD36 | CD36 molecule (thrombospondin receptor) | 7q11.2 |
| 960 | CD44 | CD44 molecule (Indian blood group) | 11p13 |
| 961 | CD47 | CD47 molecule | 3q13.1-q13.2 |
| 1030 | CDKN2B | cyclin-dependent kinase inhibitor 2B (p15, inhibits CDK4) | 9p21 |
| 1101 | CHAD | chondroadherin | 17q21.33 |
| 1277 | COL1A1 | collagen, type I, alpha 1 | 17q21.33 |
| 1278 | COL1A2 | collagen, type I, alpha 2 | 7q22.1 |
| 1280 | COL2A1 | collagen, type II, alpha 1 | 12q13.11 |
| 1281 | COL3A1 | collagen, type III, alpha 1 | 2q31 |
| 1282 | COL4A1 | collagen, type IV, alpha 1 | 13q34 |
| 1284 | COL4A2 | collagen, type IV, alpha 2 | 13q34 |
| 1286 | COL4A4 | collagen, type IV, alpha 4 | 2q35-q37 |
| 1288 | COL4A6 | collagen, type IV, alpha 6 | Xq22 |
| 1289 | COL5A1 | collagen, type V, alpha 1 | 9q34.2-q34.3 |
| 1290 | COL5A2 | collagen, type V, alpha 2 | 2q14-q32 |
| 1291 | COL6A1 | collagen, type VI, alpha 1 | 21q22.3 |
| 1292 | COL6A2 | collagen, type VI, alpha 2 | 21q22.3 |
| 1293 | COL6A3 | collagen, type VI, alpha 3 | 2q37 |
| 1301 | COL11A1 | collagen, type XI, alpha 1 | 1p21 |
| 1311 | COMP | cartilage oligomeric matrix protein | 19p13.1 |
| 1374 | CPT1A | carnitine palmitoyltransferase 1A (liver) | 11q13.1-q13.2 |
| 1375 | CPT1B | carnitine palmitoyltransferase 1B (muscle) | 22q13.33 |
| 1376 | CPT2 | carnitine palmitoyltransferase II | 1p32 |
| 1387 | CREBBP | CREB binding protein | 16p13.3 |
| 1452 | CSNK1A1 | casein kinase 1, alpha 1 | 5q32 |
| 1454 | CSNK1E | casein kinase 1, epsilon | 22q13.1 |
| 1457 | CSNK2A1 | casein kinase 2, alpha 1 polypeptide | 20p13 |
| 1459 | CSNK2A2 | casein kinase 2, alpha prime polypeptide | 16q21 |
| 1460 | CSNK2B | casein kinase 2, beta polypeptide | 6p21-p12 |
| 1462 | VCAN | versican | 5q14.3 |
| 1474 | CST6 | cystatin E/M | 11q13 |
| 1487 | CTBP1 | C-terminal binding protein 1 | 4p16 |
| 1488 | CTBP2 | C-terminal binding protein 2 | 10q26.13 |
| 1499 | CTNNB1 | catenin (cadherin-associated protein), beta 1, 88kDa | 3p21 |
| 1509 | CTSD | cathepsin D | 11p15.5 |
| 1512 | CTSH | cathepsin H | 15q24-q25 |
| 1520 | CTSS | cathepsin S | 1q21 |
| 1522 | CTSZ | cathepsin Z | 20q13 |
| 1544 | CYP1A2 | cytochrome P450, family 1, subfamily A, polypeptide 2 | 15q24.1 |
| 1548 | CYP2A6 | cytochrome P450, family 2, subfamily A, polypeptide 6 | 19q13.2 |
| 1549 | CYP2A7 | cytochrome P450, family 2, subfamily A, polypeptide 7 | 19q13.2 |
| 1553 | CYP2A13 | cytochrome P450, family 2, subfamily A, polypeptide 13 | 19q13.2 |
| 1579 | CYP4A11 | cytochrome P450, family 4, subfamily A, polypeptide 11 | 1p33 |
| 1581 | CYP7A1 | cytochrome P450, family 7, subfamily A, polypeptide 1 | 8q11-q12 |
| 1582 | CYP8B1 | cytochrome P450, family 8, subfamily B, polypeptide 1 | 3p22-p21.3 |
| 1593 | CYP27A1 | cytochrome P450, family 27, subfamily A, polypeptide 1 | 2q33-qter |
| 1605 | DAG1 | dystroglycan 1 (dystrophin-associated glycoprotein 1) | 3p21 |
| 1622 | DBI | diazepam binding inhibitor (GABA receptor modulator, acyl-Coenzyme A binding protein) | 2q12-q21 |
| 1634 | DCN | decorin | 12q21.33 |
| 1634 | DCN | decorin | 12q21.33 |
| 1805 | DPT | dermatopontin | 1q12-q23 |
| 1855 | DVL1 | dishevelled, dsh homolog 1 (Drosophila) | 1p36 |
| 1856 | DVL2 | dishevelled, dsh homolog 2 (Drosophila) | 17p13.2 |
| 1857 | DVL3 | dishevelled, dsh homolog 3 (Drosophila) | 3q27 |
| 1874 | E2F4 | E2F transcription factor 4, p107/p130-binding | 16q21-q22 |
| 1875 | E2F5 | E2F transcription factor 5, p130-binding | 8q21.2 |
| 1962 | EHHADH | enoyl-Coenzyme A, hydratase/3-hydroxyacyl Coenzyme A dehydrogenase | 3q26.3-q28 |
| 2006 | ELN | elastin | 7q11.23 |
| 2033 | EP300 | E1A binding protein p300 | 22q13.2 |
| 2033 | EP300 | E1A binding protein p300 | 22q13.2 |
| 2131 | EXT1 | exostoses (multiple) 1 | 8q24.11-q24.13 |
| 2132 | EXT2 | exostoses (multiple) 2 | 11p12-p11 |
| 2134 | EXTL1 | exostoses (multiple)-like 1 | 1p36.1 |
| 2135 | EXTL2 | exostoses (multiple)-like 2 | 1p21 |
| 2137 | EXTL3 | exostoses (multiple)-like 3 | 8p21 |
| 2167 | FABP4 | fatty acid binding protein 4, adipocyte | 8q21 |
| 2168 | FABP1 | fatty acid binding protein 1, liver | 2p11 |
| 2169 | FABP2 | fatty acid binding protein 2, intestinal | 4q28-q31 |
| 2170 | FABP3 | fatty acid binding protein 3, muscle and heart (mammary-derived growth inhibitor) | 1p33-p32 |
| 2171 | FABP5 | fatty acid binding protein 5 (psoriasisassociated) | 8q21.13 |
| 2172 | FABP6 | fatty acid binding protein 6, ileal | 5q33.3-q34 |
| 2173 | FABP7 | fatty acid binding protein 7, brain | 6q22-q23 |
| 2180 | ACSL1 | acyl-CoA synthetase long-chain family member 1 | 4q34-q35 |
| 2181 | ACSL3 | acyl-CoA synthetase long-chain family member 3 | 2q34-q35 |
| 2182 | ACSL4 | acyl-CoA synthetase long-chain family member 4 | Xq22.3-q23 |
| 2331 | FMOD | fibromodulin | 1q32 |
| 2335 | FN1 | fibronectin 1 | 2q34 |
| 2530 | FUT8 | fucosyltransferase 8 (alpha (1,6) fucosyltransferase) | 14q24.3 |
| 2535 | FZD2 | frizzled homolog 2 (Drosophila) | 17q21.1 |
| 2683 | B4GALT1 | UDP-Gal:betaGlcNAc beta 1,4-galactosyltransferase, polypeptide 1 | 9p13 |
| 2710 | GK | glycerol kinase | Xp21.3 |
| 2712 | GK2 | glycerol kinase 2 | 4q13 |
| 2713 | GK3P | glycerol kinase 3 pseudogene | 4q32.1 |
| 2811 | GP1BA | glycoprotein Ib (platelet), alpha polypeptide | 17pter-p12 |
| 2812 | GP1BB | glycoprotein Ib (platelet), beta polypeptide | 22q11.21q11.23 |
| 2814 | GP5 | glycoprotein V (platelet) | 3q29 |
| 2815 | GP9 | glycoprotein IX (platelet) | 3q21.3 |
| 2817 | GPC1 | glypican 1 | 2q35-q37 |
| 2932 | GSK3B | glycogen synthase kinase 3 beta | 3q13.3 |
| 3026 | HABP2 | hyaluronan binding protein 2 | 10q25.3 |
| 3036 | HAS1 | hyaluronan synthase 1 | 19q13.4 |
| 3037 | HAS2 | hyaluronan synthase 2 | 8q24.12 |
| 3038 | HAS3 | hyaluronan synthase 3 | 16q22.1 |
| 3158 | HMGCS2 | 3-hydroxy-3-methylglutaryl-Coenzyme A synthase 2 (mitochondrial) | 1p13-p12 |
| 3161 | HMMR | hyaluronan-mediated motility receptor (RHAMM) | 5q33.2-qter |
| 3339 | HSPG2 | heparan sulfate proteoglycan 2 | 1p36.1-p34 |
| 3339 | HSPG2 | heparan sulfate proteoglycan 2 | 1p36.1-p34 |
| 3340 | NDST1 | N-deacetylase/N-sulfotransferase (heparan glucosaminyl) 1 | 5q33.1 |
| 3371 | TNC | tenascin C | 9q33 |
| 3381 | IBSP | integrin-binding sialoprotein | 4q21-q25 |
| 3383 | ICAM1 | intercellular adhesion molecule 1 | 19p13.3-p13.2 |
| 3397 | ID1 | inhibitor of DNA binding 1, dominant negative helix-loop-helix protein | 20q11 |
| 3398 | ID2 | inhibitor of DNA binding 2, dominant negative helix-loop-helix protein | 2p25 |
| 3399 | ID3 | inhibitor of DNA binding 3, dominant negative helix-loop-helix protein | 1p36.13-p36.12 |
| 3400 | ID4 | inhibitor of DNA binding 4, dominant negative helix-loop-helix protein | 6p22-p21 |
| 3458 | IFNG | interferon, gamma | 12q14 |
| 3569 | IL6 | interleukin 6 (interferon, beta 2) | 7p21 |
| 3611 | ILK | integrin-linked kinase | 11p15.5-p15.4 |
| 3624 | INHBA | inhibin, beta A | 7p15-p13 |
| 3625 | INHBB | inhibin, beta B | 2cen-q13 |
| 3626 | INHBC | inhibin, beta C | 12q13.1 |
| 3655 | ITGA6 | integrin, alpha 6 | 2q31.1 |
| 3672 | ITGA1 | integrin, alpha 1 | 5q11.2 |
| 3673 | ITGA2 | integrin, alpha 2 (CD49B, alpha 2 subunit of VLA-2 receptor) | 5q23-q31 |
| 3674 | ITGA2B | integrin, alpha 2b (platelet glycoprotein IIb of IIb/IIIa complex, antigen CD41) | 17q21.32 |
| 3675 | ITGA3 | integrin, alpha 3 (antigen CD49C, alpha 3 subunit of VLA-3 receptor) | 17q21.33 |
| 3676 | ITGA4 | integrin, alpha 4 (antigen CD49D, alpha 4 subunit of VLA-4 receptor) | 2q31.3 |
| 3678 | ITGA5 | integrin, alpha 5 (fibronectin receptor, alpha polypeptide) | 12q11-q13 |
| 3679 | ITGA7 | integrin, alpha 7 | 12q13 |
| 3680 | ITGA9 | integrin, alpha 9 | 3p21.3 |
| 3685 | ITGAV | integrin, alpha V (vitronectin receptor, alpha polypeptide, antigen CD51) | 2q31-q32 |
| 3688 | ITGB1 | integrin, beta 1 (fibronectin receptor, beta polypeptide, antigen CD29 includes MDF2, MSK12) | 10p11.2 |
| 3690 | ITGB3 | integrin, beta 3 (platelet glycoprotein IIIa, antigen CD61) | 17q21.32 |
| 3691 | ITGB4 | integrin, beta 4 | 17q25 |
| 3693 | ITGB5 | integrin, beta 5 | 3q21.2 |
| 3694 | ITGB6 | integrin, beta 6 | 2q24.2 |
| 3695 | ITGB7 | integrin, beta 7 | 12q13.13 |
| 3696 | ITGB8 | integrin, beta 8 | 7p15.3 |
| 3725 | JUN | jun oncogene | 1p32-p31 |
| 3908 | LAMA2 | laminin, alpha 2 | 6q22-q23 |
| 3909 | LAMA3 | laminin, alpha 3 | 18q11.2 |
| 3910 | LAMA4 | laminin, alpha 4 | 6q21 |
| 3911 | LAMA5 | laminin, alpha 5 | 20q13.2-q13.3 |
| 3912 | LAMB1 | laminin, beta 1 | 7q22 |
| 3913 | LAMB2 | laminin, beta 2 (laminin S) | 3p21 |
| 3914 | LAMB3 | laminin, beta 3 | 1q32 |
| 3915 | LAMC1 | laminin, gamma 1 (formerly LAMB2) | 1q31 |
| 3918 | LAMC2 | laminin, gamma 2 | 1q25-q31 |
| 4023 | LPL | lipoprotein lipase | 8p22 |
| 4040 | LRP6 | low density lipoprotein receptor-related protein 6 | 12p11-p13 |
| 4041 | LRP5 | low density lipoprotein receptor-related protein 5 | 11q13.4 |
| 4052 | LTBP1 | latent transforming growth factor beta binding protein 1 | 2p22-p21 |
| 4053 | LTBP2 | latent transforming growth factor beta binding protein 2 | 14q24 |
| 4054 | LTBP3 | latent transforming growth factor beta binding protein 3 | 11q12 |
| 4060 | LUM | lumican | 12q21.3-q22 |
| 4086 | SMAD1 | SMAD family member 1 | 4q31 |
| 4087 | SMAD2 | SMAD family member 2 | 18q21.1 |
| 4088 | SMAD3 | SMAD family member 3 | 15q22.33 |
| 4089 | SMAD4 | SMAD family member 4 | 18q21.1 |
| 4089 | SMAD4 | SMAD family member 4 | 18q21.1 |
| 4090 | SMAD5 | SMAD family member 5 | 5q31 |
| 4091 | SMAD6 | SMAD family member 6 | 15q21-q22 |
| 4092 | SMAD7 | SMAD family member 7 | 18q21.1 |
| 4093 | SMAD9 | SMAD family member 9 | 13q12-q14 |
| 4146 | MATN1 | matrilin 1, cartilage matrix protein | 1p35 |
| 4147 | MATN2 | matrilin 2 | 8q22 |
| 4148 | MATN3 | matrilin 3 | 2p24-p23 |
| 4166 | CHST6 | carbohydrate (N-acetylglucosamine 6-O) sulfotransferase 6 | 16q22 |
| 4199 | ME1 | malic enzyme 1, NADP(+)-dependent, cytosolic | 6q12 |
| 4237 | MFAP2 | microfibrillar-associated protein 2 | 1p36.1-p35 |
| 4312 | MMP1 | matrix metallopeptidase 1 (interstitial collagenase) | 11q22.3 |
| 4313 | MMP2 | matrix metallopeptidase 2 (gelatinase A, 72kDa gelatinase, 72kDa type IV collagenase) | 16q13-q21 |
| 4314 | MMP3 | matrix metallopeptidase 3 (stromelysin 1, progelatinase) | 11q22.3 |
| 4316 | MMP7 | matrix metallopeptidase 7 (matrilysin, uterine) | 11q21-q22 |
| 4316 | MMP7 | matrix metallopeptidase 7 (matrilysin, uterine) | 11q21-q22 |
| 4319 | MMP10 | matrix metallopeptidase 10 (stromelysin 2) | 11q22.3 |
| 4320 | MMP11 | matrix metallopeptidase 11 (stromelysin 3) | 22q11.2 |
| 4321 | MMP12 | matrix metallopeptidase 12 (macrophage elastase) | 11q22.3 |
| 4322 | MMP13 | matrix metallopeptidase 13 (collagenase 3) | 11q22.3 |
| 4323 | MMP14 | matrix metallopeptidase 14 (membraneinserted) | 14q11-q12 |
| 4324 | MMP15 | matrix metallopeptidase 15 (membraneinserted) | 16q13-q21 |
| 4609 | MYC | v-myc myelocytomatosis viral oncogene homolog (avian) | 8q24.21 |
| 4772 | NFATC1 | nuclear factor of activated T-cells, cytoplasmic, calcineurin-dependent 1 | 18q23 |
| 4773 | NFATC2 | nuclear factor of activated T-cells, cytoplasmic, calcineurin-dependent 2 | 20q13.2-q13.3 |
| 4775 | NFATC3 | nuclear factor of activated T-cells, cytoplasmic, calcineurin-dependent 3 | 16q22.2 |
| 4776 | NFATC4 | nuclear factor of activated T-cells, cytoplasmic, calcineurin-dependent 4 | 14q11.2 |
| 4838 | NODAL | nodal homolog (mouse) | 10q22.1 |
| 4973 | OLR1 | oxidized low density lipoprotein (lectin-like) receptor 1 | 12p13.2-p12.3 |
| 4982 | TNFRSF11B | tumor necrosis factor receptor superfamily, member 11b | 8q24 |
| 5054 | SERPINE1 | serpin peptidase inhibitor, clade E (nexin, plasminogen activator inhibitor type 1), member 1 | 7q21.3-q22 |
| 5055 | SERPINB2 | serpin peptidase inhibitor, clade B (ovalbumin), member 2 | 18q21.3 |
| 5105 | PCK1 | phosphoenolpyruvate carboxykinase 1 (soluble) | 20q13.31 |
| 5106 | PCK2 | phosphoenolpyruvate carboxykinase 2 (mitochondrial) | 14q12 |
| 5170 | PDPK1 | 3-phosphoinositide dependent protein kinase-1 | 16p13.3 |
| 5308 | PITX2 | paired-like homeodomain 2 | 4q25-q27 |
| 5327 | PLAT | plasminogen activator, tissue | 8p12 |
| 5330 | PLCB2 | phospholipase C, beta 2 | 15q15 |
| 5331 | PLCB3 | phospholipase C, beta 3 (phosphatidylinositolspecific) | 11q13 |
| 5332 | PLCB4 | phospholipase C, beta 4 | 20p12 |
| 5346 | PLIN | perilipin | 15q26 |
| 5360 | PLTP | phospholipid transfer protein | 20q12-q13.1 |
| 5465 | PPARA | peroxisome proliferator-activated receptor alpha | 22q12-q13.1 |
| 5467 | PPARD | peroxisome proliferator-activated receptor delta | 6p21.2-p21.1 |
| 5468 | PPARG | peroxisome proliferator-activated receptor gamma | 3p25 |
| 5515 | PPP2CA | protein phosphatase 2 (formerly 2A), catalytic subunit, alpha isoform | 5q31.1 |
| 5516 | PPP2CB | protein phosphatase 2 (formerly 2A), catalytic subunit, beta isoform | 8p12 |
| 5518 | PPP2R1A | protein phosphatase 2 (formerly 2A), regulatory subunit A, alpha isoform | 19q13.33 |
| 5519 | PPP2R1B | protein phosphatase 2 (formerly 2A), regulatory subunit A, beta isoform | 11q23.2 |
| 5525 | PPP2R5A | protein phosphatase 2, regulatory subunit B', alpha isoform | 1q32.2-q32.3 |
| 5526 | PPP2R5B | protein phosphatase 2, regulatory subunit B', beta isoform | 11q12-q13 |
| 5527 | PPP2R5C | protein phosphatase 2, regulatory subunit B', gamma isoform | 14q32 |
| 5528 | PPP2R5D | protein phosphatase 2, regulatory subunit B', delta isoform | 6p21.1 |
| 5529 | PPP2R5E | protein phosphatase 2, regulatory subunit B', epsilon isoform | 14q23.1 |
| 5530 | PPP3CA | protein phosphatase 3 (formerly 2B), catalytic subunit, alpha isoform | 4q21-q24 |
| 5532 | PPP3CB | protein phosphatase 3 (formerly 2B), catalytic subunit, beta isoform | 10q21-q22 |
| 5533 | PPP3CC | protein phosphatase 3 (formerly 2B), catalytic subunit, gamma isoform | 8p21.3 |
| 5534 | PPP3R1 | protein phosphatase 3 (formerly 2B), regulatory subunit B, alpha isoform | 2p15 |
| 5535 | PPP3R2 | protein phosphatase 3 (formerly 2B), regulatory subunit B, beta isoform | 9q31.1 |
| 5566 | PRKACA | protein kinase, cAMP-dependent, catalytic, alpha | 19p13.1 |
| 5567 | PRKACB | protein kinase, cAMP-dependent, catalytic, beta | 1p36.1 |
| 5568 | PRKACG | protein kinase, cAMP-dependent, catalytic, gamma | 9q13 |
| 5578 | PRKCA | protein kinase C, alpha | 17q22-q23.2 |
| 5579 | PRKCB | protein kinase C, beta | 16p11.2 |
| 5582 | PRKCG | protein kinase C, gamma | 19q13.4 |
| 5594 | MAPK1 | mitogen-activated protein kinase 1 | 22q 11.2 |
| 5595 | MAPK3 | mitogen-activated protein kinase 3 | 16p11.2 |
| 5599 | MAPK8 | mitogen-activated protein kinase 8 | 10q11.22 |
| 5601 | MAPK9 | mitogen-activated protein kinase 9 | 5q35 |
| 5602 | MAPK10 | mitogen-activated protein kinase 10 | 4q22.1-q23 |
| 5613 | PRKX | protein kinase, X-linked | Xp22.3 |
| 5616 | PRKY | protein kinase, Y-linked | Yp11.2 |
| 5649 | RELN | reelin | 7q22 |
| 5663 | PSEN1 | presenilin 1 | 14q24.3 |
| 5879 | RAC1 | ras-related C3 botulinum toxin substrate 1 (rho family, small GTP binding protein Rac1) | 7p22 |
| 5880 | RAC2 | ras-related C3 botulinum toxin substrate 2 (rho family, small GTP binding protein Rac2) | 22q13.1 |
| 5881 | RAC3 | ras-related C3 botulinum toxin substrate 3 (rho family, small GTP binding protein Rac3) | 17q25.3 |
| 5933 | RBL1 | retinoblastoma-like 1 (p107) | 20q11.2 |
| 5934 | RBL2 | retinoblastoma-like 2 (p130) | 16q12.2 |
| 5950 | RBP4 | retinol binding protein 4, plasma | 10q23-q24 |
| 6093 | ROCK1 | Rho-associated, coiled-coil containing protein kinase 1 | 18q11.1 |
| 6198 | RPS6KB1 | ribosomal protein S6 kinase, 70kDa, polypeptide 1 | 17q23.1 |
| 6199 | RPS6KB2 | ribosomal protein S6 kinase, 70kDa, polypeptide 2 | 11q13.1 |
| 6256 | RXRA | retinoid X receptor, alpha | 9q34.3 |
| 6257 | RXRB | retinoid X receptor, beta | 6p21.3 |
| 6258 | RXRG | retinoid X receptor, gamma | 1q22-q23 |
| 6319 | SCD | stearoyl-CoA desaturase (delta-9-desaturase) | 10q24.31 |
| 6342 | SCP2 | sterol carrier protein 2 | 1p32 |
| 6382 | SDC1 | syndecan 1 | 2p24.1 |
| 6383 | SDC2 | syndecan 2 | 8q22-q23 |
| 6385 | SDC4 | syndecan 4 | 20q12 |
| 6422 | SFRP1 | secreted frizzled-related protein 1 | 8p12-p11.1 |
| 6423 | SFRP2 | secreted frizzled-related protein 2 | 4q31.3 |
| 6424 | SFRP4 | secreted frizzled-related protein 4 | 7p14.1 |
| 6425 | SFRP5 | secreted frizzled-related protein 5 | 10q24.1 |
| 6477 | SIAH1 | seven in absentia homolog 1 (Drosophila) | 16q12 |
| 6482 | ST3GAL1 | ST3 beta-galactoside alpha-2,3sialyltransferase 1 | 8q24.22 |
| 6483 | ST3GAL2 | ST3 beta-galactoside alpha-2,3sialyltransferase 2 | 16q22.1 |
| 6487 | ST3GAL3 | ST3 beta-galactoside alpha-2,3sialyltransferase 3 | 1p34.1 |
| 6500 | SKP1 | S-phase kinase-associated protein 1 | 5q31 |
| 6667 | SP1 | Sp1 transcription factor | 12q13.1 |
| 6696 | SPP1 | secreted phosphoprotein 1 | 4q21-q25 |
| 6885 | MAP3K7 | mitogen-activated protein kinase kinase kinase 7 | 6q16.1-q16.3 |
| 6907 | TBL1X | transducin (beta)-like 1X-linked | Xp22.3 |
| 6932 | TCF7 | transcription factor 7 (T-cell specific, HMG-box) | 5q31.1 |
| 6934 | TCF7L2 | transcription factor 7-like 2 (T-cell specific, HMG-box) | 10q25.3 |
| 7027 | TFDP1 | transcription factor Dp-1 | 13q34 |
| 7040 | TGFB1 | transforming growth factor, beta 1 | 19q13.2 |
| 7042 | TGFB2 | transforming growth factor, beta 2 | 1q41 |
| 7043 | TGFB3 | transforming growth factor, beta 3 | 14q24 |
| 7044 | LEFTY2 | left-right determination factor 2 | 1q42.1 |
| 7046 | TGFBR1 | transforming growth factor, beta receptor 1 | 9q22 |
| 7048 | TGFBR2 | transforming growth factor, beta receptor II (70/80kDa) | 3p22 |
| 7057 | THBS1 | thrombospondin 1 | 15q15 |
| 7058 | THBS2 | thrombospondin 2 | 6q27 |
| 7059 | THBS3 | thrombospondin 3 | 1q21 |
| 7060 | THBS4 | thrombospondin 4 | 5q13 |
| 7076 | TIMP1 | TIMP metallopeptidase inhibitor 1 | Xp11.3-p11.23 |
| 7077 | TIMP2 | TIMP metallopeptidase inhibitor 2 | 17q25 |
| 7124 | TNF | tumor necrosis factor (TNF superfamily, member 2) | 6p21.3 |
| 7143 | TNR | tenascin R (restrictin, janusin) | 1q24 |
| 7146 | TNXA | tenascin XA pseudogene | 6p21.3 |
| 7148 | TNXB | tenascin XB | 6p21.3 |
| 7157 | TP53 | tumor protein p53 | 17p13.1 |
| 7316 | UBC | ubiquitin C | 12q24.3 |
| 7350 | UCP1 | uncoupling protein 1 (mitochondrial, proton carrier) | 4q28-q31 |
| 7448 | VTN | vitronectin | 17q11 |
| 7450 | VWF | von Willebrand factor | 12p13.3 |
| 7471 | WNT1 | wingless-type MMTV integration site family, member 1 | 12q13 |
| 7472 | WNT2 | wingless-type MMTV integration site family member 2 | 7q31.2 |
| 7473 | WNT3 | wingless-type MMTV integration site family, member 3 | 17q21 |
| 7474 | WNT5A | wingless-type MMTV integration site family, member 5A | 3p21-p14 |
| 7475 | WNT6 | wingless-type MMTV integration site family, member 6 | 2q35 |
| 7476 | WNT7A | wingless-type MMTV integration site family, member 7A | 3p25 |
| 7477 | WNT7B | wingless-type MMTV integration site family, member 7B | 22q13 |
| 7478 | WNT8A | wingless-type MMTV integration site family, member 8A | 5q31 |
| 7479 | WNT8B | wingless-type MMTV integration site family, member 8B | 10q24 |
| 7480 | WNT10B | wingless-type MMTV integration site family, member 10B | 12q13 |
| 7481 | WNT11 | wingless-type MMTV integration site family, member 11 | 11q13.5 |
| 7482 | WNT2B | wingless-type MMTV integration site family, member 2B | 1p13 |
| 7483 | WNT9A | wingless-type MMTV integration site family, member 9A | 1q42 |
| 7484 | WNT9B | wingless-type MMTV integration site family, member 9B | 17q21 |
| 7498 | XDH | xanthine dehydrogenase | 2p23.1 |
| 7839 | LSL | Leptin, serum levels of | 2p21 |
| 7855 | FZD5 | frizzled homolog 5 (Drosophila) | 2q33-q34 |
| 7976 | FZD3 | frizzled homolog 3 (Drosophila) | 8p21 |
| 8061 | FOSL1 | FOS-like antigen 1 | 11q13 |
| 8200 | GDF5 | growth differentiation factor 5 | 20q11.2 |
| 8215 | DVL1L1 | dishevelled, dsh homolog 1 (Drosophila)-like 1 | 22q11.21 |
| 8309 | ACOX2 | acyl-Coenzyme A oxidase 2, branched chain | 3p14.3 |
| 8310 | ACOX3 | acyl-Coenzyme A oxidase 3, pristanoyl | 4p15.3 |
| 8312 | AXIN1 | axin 1 | 16p13.3 |
| 8313 | AXIN2 | axin 2 | 17q23-q24 |
| 8321 | FZD1 | frizzled homolog 1 (Drosophila) | 7q21 |
| 8322 | FZD4 | frizzled homolog 4 (Drosophila) | 11q14.2 |
| 8323 | FZD6 | frizzled homolog 6 (Drosophila) | 8q22.3-q23.1 |
| 8324 | FZD7 | frizzled homolog 7 (Drosophila) | 2q33 |
| 8325 | FZD8 | frizzled homolog 8 (Drosophila) | 10p11.21 |
| 8326 | FZD9 | frizzled homolog 9 (Drosophila) | 7q11.23 |
| 8425 | LTBP4 | latent transforming growth factor beta binding protein 4 | 19q13.1-q13.2 |
| 8454 | CUL1 | cullin 1 | 7q36.1 |
| 8509 | NDST2 | N-deacetylase/N-sulfotransferase (heparan glucosaminyl) 2 | 10q22 |
| 8515 | ITGA10 | integrin, alpha 10 | 1q21 |
| 8516 | ITGA8 | integrin, alpha 8 | 10p13 |
| 8534 | CHST1 | carbohydrate (keratan sulfate Gal-6) sulfotransferase 1 | 11p11.2-p11.1 |
| 8607 | RUVBL1 | RuvB-like 1 (E. coli) | 3q21 |
| 8646 | CHRD | chordin | 3q27 |
| 8649 | MAPKSP1 | MAPK scaffold protein 1 | 4q23 |
| 8702 | B4GALT4 | UDP-Gal:betaGlcNAc beta 1,4-galactosyltransferase, polypeptide 4 | 3q13.3 |
| 8703 | B4GALT3 | UDP-Gal:betaGlcNAc beta 1,4-galactosyltransferase, polypeptide 3 | 1q21-q23 |
| 8704 | B4GALT2 | UDP-Gal:betaGlcNAc beta 1,4-galactosyltransferase, polypeptide 2 | 1p34-p33 |
| 8759 | ADAM1 | ADAM metallopeptidase domain 1 (pseudogene) | 12q24.13 |
| 8785 | MATN4 | matrilin 4 | 20q13.1-q13.2 |
| 8945 | BTRC | beta-transducin repeat containing | 10q24.32 |
| 9241 | NOG | noggin | 17q21-q22 |
| 9348 | NDST3 | N-deacetylase/N-sulfotransferase (heparan glucosaminyl) 3 | 4q26 |
| 9350 | CER1 | cerberus 1, cysteine knot superfamily, homolog (Xenopus laevis) | 9p23-p22 |
| 9370 | ADIPOQ | adiponectin, C1Q and collagen domain containing | 3q27 |
| 9372 | ZFYVE9 | zinc finger, FYVE domain containing 9 | 1p32.3 |
| 9394 | HS6ST1 | heparan sulfate 6-O-sulfotransferase 1 | 2q21 |
| 9415 | FADS2 | fatty acid desaturase 2 | 11q12-q13.1 |
| 9435 | CHST2 | carbohydrate (N-acetylglucosamine-6-O) sulfotransferase 2 | 3q24 |
| 9469 | CHST3 | carbohydrate (chondroitin 6) sulfotransferase 3 | 10q22.1 |
| 9475 | ROCK2 | Rho-associated, coiled-coil containing protein kinase 2 | 2p24 |
| 9653 | HS2ST1 | heparan sulfate 2-O-sulfotransferase 1 | 1p31.1-p22.1 |
| 9672 | SDC3 | syndecan 3 | 1pter-p22.3 |
| 9765 | ZFYVE16 | zinc finger, FYVE domain containing 16 | 5q14 |
| 9899 | SV2B | synaptic vesicle glycoprotein 2B | 15q26.1 |
| 9900 | SV2A | synaptic vesicle glycoprotein 2A | 1q21.2 |
| 9953 | HS3ST3B1 | heparan sulfate (glucosamine) 3-Osulfotransferase 3B1 | 17p12-p11.2 |
| 9955 | HS3ST3A1 | heparan sulfate (glucosamine) 3-Osulfotransferase 3A1 | 17p12-p11.2 |
| 9956 | HS3ST2 | heparan sulfate (glucosamine) 3-Osulfotransferase 2 | 16p12 |
| 9957 | HS3ST1 | heparan sulfate (glucosamine) 3-Osulfotransferase 1 | 4p16 |
| 9978 | RBX1 | ring-box 1 | 22q13.2 |
| 10023 | FRAT1 | frequently rearranged in advanced T-cell lymphomas | 10q24.1 |
| 10062 | NR1H3 | nuclear receptor subfamily 1, group H, member 3 | 11p11.2 |
| 10090 | UST | uronyl-2-sulfotransferase | 6q25.1 |
| 10135 | NAMPT | nicotinamide phosphoribosyltransferase | 7q22.2 |
| 10164 | CHST4 | carbohydrate (N-acetylglucosamine 6-O) sulfotransferase 4 | 16q22.3 |
| 10297 | APC2 | adenomatosis polyposis coli 2 | 19p13.3 |
| 10319 | LAMC3 | laminin, gamma 3 | 9q31-q34 |
| 10468 | FST | follistatin | 5q11.2 |
| 10580 | SORBS1 | sorbin and SH3 domain containing 1 | 10q23.3-q24.1 |
| 10637 | LEFTY1 | left-right determination factor 1 | 1q42.1 |
| 10678 | B3GNT2 | UDP-GlcNAc:betaGal beta-1,3-Nacetylglucosaminyltransferase 2 | 2p15 |
| 10725 | NFAT5 | nuclear factor of activated T-cells 5, tonicityresponsive | 16q22.1 |
| 10998 | SLC27A5 | solute carrier family 27 (fatty acid transporter), member 5 | 19q13.43 |
| 10999 | SLC27A4 | solute carrier family 27 (fatty acid transporter), member 4 | 9q34.11 |
| 11001 | SLC27A2 | solute carrier family 27 (fatty acid transporter), member 2 | 15q21.2 |
| 11041 | B3GNT1 | UDP-GlcNAc:betaGal beta-1,3-Nacetylglucosaminyltransferase 1 | 11q13.1 |
| 11197 | WIF1 | WNT inhibitory factor 1 | 12q14.3 |
| 11211 | FZD10 | frizzled homolog 10 (Drosophila) | 12q24.33 |
| 11285 | B4GALT7 | xylosylprotein beta 1,4-galactosyltransferase, polypeptide 7 (galactosyltransferase I) | 5q35.2-q35.3 |
| 22798 | LAMB4 | laminin, beta 4 | 7q22-q31.2 |
| 22801 | ITGA11 | integrin, alpha 11 | 15q23 |
| 22856 | CHSY1 | chondroitin sulfate synthase 1 | 15q26.3 |
| 22927 | HABP4 | hyaluronan binding protein 4 | 9q22.3-q31 |
| 22943 | DKK1 | dickkopf homolog 1 (Xenopus laevis) | 10q11.2 |
| 22987 | SV2C | synaptic vesicle glycoprotein 2C | 5q13.3 |
| 23002 | DAAM1 | dishevelled associated activator of morphogenesis 1 | 14q23.1 |
| 23236 | PLCB1 | phospholipase C, beta 1 (phosphoinositidespecific) | 20p12 |
| 23291 | FBXW11 | F-box and WD repeat domain containing 11 | 5q35.1 |
| 23305 | ACSL6 | acyl-CoA synthetase long-chain family member 6 | 5q31 |
| 23401 | FRAT2 | frequently rearranged in advanced T-cell lymphomas 2 | 10q24.1 |
| 23500 | DAAM2 | dishevelled associated activator of morphogenesis 2 | 6p21.2 |
| 26035 | GLCE | glucuronic acid epimerase | 15q23 |
| 26229 | B3GAT3 | beta-1,3-glucuronyltransferase 3 (glucuronosyltransferase I) | 11q12.3 |
| 27087 | B3GAT1 | beta-1,3-glucuronyltransferase 1 (glucuronosyltransferase P) | 11q25 |
| 27101 | CACYBP | calcyclin binding protein | 1q24-q25 |
| 27121 | DKK4 | dickkopf homolog 4 (Xenopus laevis) | 8p11.2-p11.1 |
| 27123 | DKK2 | dickkopf homolog 2 (Xenopus laevis) | 4q25 |
| 28965 | SLC27A6 | solute carrier family 27 (fatty acid transporter), member 6 | 5q23.3 |
| 29940 | DSE | dermatan sulfate epimerase | 6q22 |
| 50509 | COL5A3 | collagen, type V, alpha 3 | 19p13.2 |
| 50515 | CHST11 | carbohydrate (chondroitin 4) sulfotransferase 11 | 12q |
| 51129 | ANGPTL4 | angiopoietin-like 4 | 19p13.3 |
| 51176 | LEF1 | lymphoid enhancer-binding factor 1 | 4q23-q25 |
| 51206 | GP6 | glycoprotein VI (platelet) | 19q13.4 |
| 51384 | WNT16 | wingless-type MMTV integration site family, member 16 | 7q31 |
| 51701 | NLK | nemo-like kinase | 17q11.2 |
| 51703 | ACSL5 | acyl-CoA synthetase long-chain family member 5 | 10q25.1-q25.2 |
| 54361 | WNT4 | wingless-type MMTV integration site family, member 4 | 1p36.23-p35.1 |
| 55454 | CSGALNACT2 | chondroitin sulfate Nacetylgalactosaminyltransferase 2 | 10q11.21 |
| 55501 | CHST12 | carbohydrate (chondroitin 4) sulfotransferase 12 | 7p22 |
| 55790 | CSGALNACT1 | chondroitin sulfate Nacetylgalactosaminyltransferase 1 | 8p21.3 |
| 56548 | CHST7 | carbohydrate (N-acetylglucosamine 6-O) sulfotransferase 7 | Xp11.23 |
| 56729 | RETN | resistin | 19p13.2 |
| 56998 | CTNNBIP1 | catenin, beta interacting protein 1 | 1p36.22 |
| 57154 | SMURF1 | SMAD specific E3 ubiquitin protein ligase 1 | 7q22.1 |
| 57216 | VANGL2 | vang-like 2 (van gogh, Drosophila) | 1q22-q23 |
| 57680 | CHD8 | chromodomain helicase DNA binding protein 8 | 14q11.2 |
| 58496 | LY6G5B | lymphocyte antigen 6 complex, locus G5B | 6p21.3 |
| 59343 | SENP2 | SUMO1/sentrin/SMT3 specific peptidase 2 | 3q27.2 |
| 63923 | TNN | tenascin N | 1q23-q24 |
| 64131 | XYLT1 | xylosyltransferase I | 16p12.3 |
| 64132 | XYLT2 | xylosyltransferase II | 17q21.3-q22 |
| 64321 | SOX17 | SRY (sex determining region Y)-box 17 | 8q11.23 |
| 64579 | NDST4 | N-deacetylase/N-sulfotransferase (heparan glucosaminyl) 4 | 4q25-q26 |
| 64750 | SMURF2 | SMAD specific E3 ubiquitin protein ligase 2 | 17q22-q23 |
| 64840 | PORCN | porcupine homolog (Drosophila) | Xp11.23 |
| 79586 | CHPF | chondroitin polymerizing factor | 2q35 |
| 79718 | TBL1XR1 | transducin (beta)-like 1 X-linked receptor 1 | 3q26.32 |
| 79966 | SCD5 | stearoyl-CoA desaturase 5 | 4q21.22 |
| 80070 | ADAMTS20 | ADAM metallopeptidase with thrombospondin type 1 motif, 20 | 12q12 |
| 80319 | CXXC4 | CXXC finger 4 | 4q22-q24 |
| 80326 | WNT10A | wingless-type MMTV integration site family, member 10A | 2q35 |
| 81029 | WNT5B | wingless-type MMTV integration site family, member 5B | 12p13.3 |
| 81839 | VANGL1 | vang-like 1 (van gogh, Drosophila) | 1p11-p13.1 |
| 83439 | TCF7L1 | transcription factor 7-like 1 (T-cell specific, HMG-box) | 2p11.2 |
| 83729 | INHBE | inhibin, beta E | 12q13.3 |
| 85407 | NKD1 | naked cuticle homolog 1 (Drosophila) | 16q12 |
| 85409 | NKD2 | naked cuticle homolog 2 (Drosophila) | 5p15.3 |
| 89780 | WNT3A | wingless-type MMTV integration site family, member 3A | 1q42 |
| 90161 | HS6ST2 | heparan sulfate 6-O-sulfotransferase 2 | Xq26.2 |
| 90665 | TBL1Y | transducin (beta)-like 1Y-linked | Yp11.2 |
| 93010 | B3GNT7 | UDP-GlcNAc:betaGal beta-1,3-Nacetylglucosaminyltransferase 7 | 2q37.1 |
| 113189 | CHST14 | carbohydrate (N-acetylgalactosamine 4-0) sulfotransferase 14 | 15q15.1 |
| 116519 | APOA5 | apolipoprotein A-V | 11q23 |
| 122011 | CSNK1A1L | casein kinase 1, alpha 1-like | 13q13.3 |
| 126129 | CPT1C | carnitine palmitoyltransferase 1C | 19q13.33 |
| 126792 | B3GALT6 | UDP-Gal:betaGal beta 1,3-galactosyltransferase polypeptide 6 | 1p36.33 |
| 130399 | ACVR1C | activin A receptor, type IC | 2q24.1 |
| 135152 | B3GAT2 | beta-1,3-glucuronyltransferase 2 (glucuronosyltransferase S) | 6q13 |
| 144165 | PRICKLE1 | prickle homolog 1 (Drosophila) | 12q12 |
| 151449 | GDF7 | growth differentiation factor 7 | 2p24.1 |
| 166012 | CHST13 | carbohydrate (chondroitin 4) sulfotransferase 13 | 3q21.3 |
| 166336 | PRICKLE2 | prickle homolog 2 (Drosophila) | 3p14.1 |
| 222537 | HS3ST5 | heparan sulfate (glucosamine) 3-Osulfotransferase 5 | 6q22.31 |
| 266722 | HS6ST3 | heparan sulfate 6-O-sulfotransferase 3 | 13q32.1 |
| 283106 | CSNK2A1P | casein kinase 2, alpha 1 polypeptide pseudogene | 11p15.3 |
| 284217 | LAMA1 | laminin, alpha 1 | 18p11.31 |
| 284541 | CYP4A22 | cytochrome P450, family 4, subfamily A, polypeptide 22 | 1p33 |
| 337876 | CHSY3 | chondroitin sulfate synthase 3 | 5q23.3 |
| 353500 | BMP8A | bone morphogenetic protein 8a | 1p34.2 |
| 375790 | AGRN | agrin | 1p36.33 |
| 376497 | SLC27A1 | solute carrier family 27 (fatty acid transporter), member 1 | 19p13.11 |
| 392255 | GDF6 | growth differentiation factor 6 | 8q22.1 |
| 642956 | FABP5L9 | fatty acid binding protein 5-like 9 | 15q25.3 |
| 728641 | FABP5L7 | fatty acid binding protein 5-like 7 | 11q12.1 |
| 728729 | FABP5L8 | fatty acid binding protein 5-like 8 | 15q25.2 |
| 729163 | FABP5L2 | fatty acid binding protein 5-like 2 | 13q14.3 |

### Humane Primärzellen

Als biologische Testsysteme werden humane dermale Fibroblasten bzw. humane subcutane Präadipozyten in Form von *in vitro* Zellkultursystemen verwendet. Beide Zelltypen sind kommerziell verfügbar (Firma Lifeline oder die Firma CellApplications).

Humane dermale Fibroblasten werden im Regelfall aus der Dermis neonataler Spender (Fimosen) oder aus der Haut adulter Spender gewonnen. Es handelt sich stets um nicht infektiöses Spender- Materialien, die seitens des jeweiligen Suppliers aus dem *in vivo* Gewebeverband isoliert und einmalig in einem korrespondierenden Zellkulturmedium expandiert wurden. Cryokonservierte humane dermale Fobroblasten können nach ihrer Aussaat in geeignete Zellkulturmedien ohne Beeinträchtigung ihrer typischen Physiologie und Morphologie bis zu 16 Verdopplungszyklen durchlaufen. Humane dermale Fibroblasten kommen in allen Bindegewebsstrukturen vor und stellen ein gut charakterisiertes *in vitro* Testsystem dar. Sie setzen *in vitro* extrazelluläre Matrixproteine frei und eignen sich in der *in vitro* Forschung insbesondere auch zur Erforschung des Fibroblasten-Wachstums, der Erforschung der Differenzierung von Fibroblasten sowie insbesondere auch zur Erforschung des Kollagen-Stoffwechsels im Kontext der Wundheilung. Es konnte verschiedentlich gezeigt werden, dass sich humane, dermale Fibroblasten zur Population von *in vitro* rekonstituierten, dreidimensionalen Dermisäquivalenten und *in vitro* rekonstituierten humanen Hautmodellen eignen.

Humane Präadipozyten (HPAd) werden gewöhnlich aus humanem Fettgewebe nicht infektiöser Spender gewonnen. Während sich humane Adipozyten nicht zur Cryokonservierung und somit auch nicht zu einer evtl. Neuaussaat nach Extraktion der Zellen aus dem Spendergewebe eignen, lassen sich humane Präadipozyten nach einer korrespondierenden Extraktion sehr gut cryokonservieren und einer *in vitro* Kultivierung unterziehen. Sie lassen sich aber nicht häufiger als zweifach passagieren. Danach differenzieren sie in humane Adipozyten. Humane Präadipozyten zeichnen sich durch eine Morphologie aus, die sehr der Morphologie humaner, dermaler Fibroblasten ähnelt. Humane Adipozyten, die durch eine *in vitro* Differenzierung aus humanen Präadipozyten gewonnen wurden, lassen sich als *in vitro* Testsystem zur Erforschung des Insulin-stimulierten Glucosetransports, zur Charakterisierung der Hormon-gesteuerten Lipolyse sowie zur Erforschung der Genexpression in Fettgeweben verwenden. Das HPAd/HAd System ist ein überaus gut charakterisiertes *in vitro* Testsystem zur Erforschung der Ursachen von Adipositas und Typ II Diabetes.

### Kontaktieren der humanen Zellen mit Hefeextrakt

Humane prim. Fibroblasten wurden in Medium 199 (Souto LR, Rehder J, Vassallo J, Cintra ML, Kraemer MH, Puzzi MB (2006) Sao Paulo Med J. 124(2), 71-6. Model for human skin reconstructed in vitro composed of associated dermis and epidermis; E. Pinney, K. Liu, B. Sheeman and J. Mansbridge (2001): Human three-dimensional fibroblast cultures express angiogenic activity. J Cell Physiol 183, 74-82) bis zu 75 %iger Subkonfluenz mit 10% FCS (Fötales Kalbsserum) und 2% Penicillin (Pen)/Streptomycin (Strep) expandiert. Nach der Zellernte wurden die Zellen in die extrazelluläre Collagenmatrix (ECM) integriert und in Gegenwart von M199 Medium mit 5% FCS und 2% Pen /Strep kultiviert. Die Exposition mit dem Active (Hefeextrakt) erfolgte 4-5 Tage nach Aussaat der Zellen in die ECM über eien Zeitraum von 24 - 48h. Die Zellviabilität wurde während der Kultivierung mit Hilfe des Standard MTT Tests (Mosmann, Tim "Rapid colorimetric assay for cellular growth and survival: application to proliferation and cytotoxicity assays". *Journal Of Immunological Methods* **65** (1-2): 55-63.) kontrolliert. Es wurden normale Zellkulturbedingungen (5% CO₂; 37°C, max. Humidität) gewählt.

Humane Präadipozyten wurden unter normalen Zellkulturbedingungen und unter Verwendung eines speziellen Wachstumsmediums (Supplier: Cell Applications) bis zu einer 60 - 75 %igen Subkonfluenz expandiert. Dabei wurde die Differenzierung der Zellen zu Adipozyten inhibiert. Nach Ernte der Zellen und erneuter Aussaat in 6Well-Platten (> 10.000 Zellen/cm2) wurden die Zellen für 24 - 48 h in Gegenwart des Wachstumsmediums kultiviert und anschließend unter Verwendung eines speziellen Differenzierungsmediums (Supplier: Cell Applications) differenziert. Die Differenzierung erfolgte über einen Zeitraum von 10 - 14 Tagen in Gegenwart des Hefeextraktes ("repeated dose" kontinuierlich alle 5 - 7 Tage).

Die Zellen in Co-Kultur wurden jeweils wie oben beschrieben expandiert. 48 h nach Integration der Fibroblasten in die ECM (wie oben beschrieben) wurden die hum. Präadipozyten auf die Collagen-Matrix aufgebracht. Das gesamte Konstrukt wurde unter normalen Zellkulturbedingungen und unter Verwendung des (Prä)Adipozyten Wachstumsmediums für weitere 48h kultiviert. Danach erfolgte die Differenzierung der Präadipozyten in Gegenwart des Actives unter Verwendung des Differenzierungsmediums. Die Zellviabilität beider Zelltypen wurde wiederum mit Hilfe des Standard MTT Tests kontrolliert. Die Freisetzung intrazellulärer Laktatdehydrogenase (LDH) wurde zur Bewertung der Integrität der Zellmembranen quantitativ bestimmt.

Humane epidermale Keratinozyten wurden in Medium 154 von Cascade Biologics (Invitrogen Company) expandiert. Nach Erreichen einer 70 - 80% igen Subkonfluenz wurden die Zellen geerntet und in Gegenwart des oben beschriebenen Mediums auf einer Polycarbonatmembran ( < 0.4 µM Porengröße) über einen Zeitraum von > 48 h submers kultiviert. Das Medium wurde zusätzlich mit FCS, und einem BSA (Rinderserumalbumin):Fettsäure Komplex sowie Vitamin E; L-Serin und L-Carnitin supplementiert. Anschließend erfolgte ein Shift der Zellen an die Luft-Medium Grenzschicht und eine Umstellung auf ein Serum-freies Differenzierungsmedium mit erhöhter Ca²⁺ Ionenkonzentration unter Zugabe von Vitamin C. Die Zellen wurden ab dem 7. Tag bis zum 14. Tag dieser Airlift-Kultur in Gegenwart des Actives kultiviert ("repeated dose" kontinuierlich, täglich). Auch in diesem Falle wurden normale Zellkulturbedingungen gewählt.

### Isolierung der RNA aus den Fibroblasten

Die Isolierung der gesamt-RNA aus den Fibroblasten wird mittels des RNeasy Mini Kits durchgeführt. Zur Analyse werden die eingefrorenen Fibroblasten mit einer 5 mm Stahlkugel, 300 µl RLT Puffer und mit 10 µl/ml beta Mercaptoethanol im Tissuelyser für 3 min bei 16.000 Hertz homogenisiert. Die Proben werden kurz anzentrifugiert, um den Schaum im Eppendorf-Cap nach unten zubekommen. Die Lösung wird für mit 590 µl RNase freiem Wasser und 10 µl Protease K versetzt und 10 min bei 55°C im Thermo-Schüttler verdaut. Der Überstand von ca. 900 µl wird in ein neues Eppendorf-Cap gegeben und mit 450 µl 96%igem Ethanol versetzt. Es werden 700 µL abgenommen, auf eine RNeasy Mini Spin Column überführt und 15 s bei 10000 U/min bei Raumtemperatur zentrifugiert, die Flüssigkeit kann verworfen werden. Diesen Schritt mit der verbleibenden Flüssigkeit wiederholen. Anschließend werden 700 µL Puffer RW1 auf die RNeasy Mini Spin Column gegeben und bei Raumtemperatur 15 s bei 10.000 U/min zentrifugiert, die Flüssigkeit kann verworfen werden. Die RNeasy Mini Spin Column werden auf ein neues Tube (2,2 ml) gegeben und mit 500 µL Puffer RPE versetzt und erneut 15 s bei 10000 U/min zentrifugiert, die Flüssigkeit wird verworfen. Nun werden 500 µL Puffer RPE zugegeben, und bei Raumtemperatur 2 min bei 10.000 U/min zentrifugiert, die Flüssigkeit wird verworfen. Die RNeasy Mini Spin Column werden auf ein neues Tube (2,2 ml) gegeben und bei Raumtemperatur 1 min bei 13.000 U/min trocken zentrifugiert. Die RNeasy Mini Spin Column werden auf ein Eppendorf-Cap (1,5 ml) gegeben und 30 µl RNase freiem Wasser direkt auf die Membrane gegeben. Anschließend für 1 min bei 10.000 U/min zentrifugiert. Zur Messung werden 3 µL RNA-Lösung am Bioanalyser untersucht. Die restliche Lösung wird aufgeteilt (je 5 µL) und im Gefrierschrank bei -80 °C gelagert.

### Isolierung der RNA aus den Adipozyten

Die Isolierung der Gesamt-RNA aus den Adipozyten wird mittels des RNeasy Mini Kits durchgeführt. Dazu wird das Differenzierungs-/Expositionsmedium durch Dekantieren quantitativ von den Adipozyten entfernt. Zur Lyse der Zellen und Freisetzung der cytosolischen RNA werden die Adipozyten in den 6-Well Zellkulturplatten über einen Zeitraum von 2 min bei RT in Gegenwart eines Guanidinium-Hydrochlorid haltigen Puffers (V = 600 µL; Qiagen Puffer RLT) inkubiert. Zum vollständigen Aufschluss der Zellen wird die Zellsuspension mit Hilfe einer 1 mL Einmalspritze und einer Einmal-Injektions-Kanüle (0,60 x 30 mm; 23 G x 1 ¼) geschert. Der zellfreie Extrakt wird quantitativ in ein 2 mL Reaktionsgefäß überführt. Die Gesamt-RNA wird durch Zugabe eines Volumens Ethanol (70 %)gefällt. Im Anschluss werden 700 µL des gefällten Lysates, auf eine RNeasy mini column überführt und 15 s bei ≥ 8000 x g zentrifugiert. Dabei wird die Gesamt-RNA an das Säulenmaterial gebunden. Der Durchfluss wird verworfen. Mit dem verbleibende Volumen des Lysates wird in gleicher Weise verfahren. Anschließend werden 700 µL eines Waschpuffers (Qiagen Puffer RW1) zugegeben und 15 s bei ≥ 8000 x g zentrifugiert. Der Durchfluss wird wiederum verworfen. Im Anschluss werden die Säulen zweimalig mit 500 µL eines Ethanol haltigen Puffers (Qiagen Puffer RPE) gewaschen. Der erste Waschvorgang erfolgt durch Zentrifugation über einen Zeitraum von 15 s bei ≥ 8000 x g (Durchfluss wird verworfen) und der zweite Waschvorgang erfolgt über einen Zeitraum von 2 min bei ≥ 8000 x g (Durchfluss wird wiederum verworfen). Zur vollständigen Trocknung des Säulenmaterials werden die Extraktionssäulen in neue Sammelgefäße überführt und über einen Zeitraum von 1 min bei 8000 x g zentrifugiert. Die Extraktionssäulen werden anschließend in 1,5 ml Reaktionsgefäße überführt und die auf den Säulen gebundene gesamt RNA wird durch Zugabe von 50 µL Wasser (RNase-free) rehydratisiert. Die finale Elution der Gesamt-RNA erfolgt anschließend durch einen weiteren Zentrifugationsschritt (1 min bei > 8000 x g). Zur Messung werden 10 µL RNA-Lösung mit 90 µL Wasser versetzt und die Absorption bei einer Wellenlänge von 260 nm gemessen. Eine Absorption (A260) von 1,00 entspricht einer Konzenztration von 50 µg/ml doppelsträngiger DNA, 33 µg/ml kurzer, einzelsträngiger DNA oder 40 µg/ml RNA. Die restlichen 40 µL Lösung werden aufgeteilt (je 10 µL) und im Gefrierschrank bei -80 °C gelagert.

### Chiptechnologie

Die isolierte mRNA wird mittels Biochip untersucht. Für diesen Biochip wurden 500 Biomarker (siehe **Tab. 1)** ausgewählt und deren spezifischer Pathway unersucht. Der Biochip wurde entwickelt von der Firma Febit Biomed GmbH in Heidelberg. Für jeden Biomarker wurden fünf unterschiedliche Proben und für jede Probe sechs technische Replikate erzeugt, so dass für jeden Biomarker 30 Replikate existieren. Der Geniom Biochip wurde synthetisiert mit dem Geniom One Instrument unter Verwendung des Standard Kits für Oligonucleotide Synthesen. Die lichtaktivierten in-situ Oligonucleotidsynthese wird mit einem digital Micromirror Gerät mit dem Geniom One Instrument an einem activierten dreidimentionalen Reaktionsüberträger auf einer glass-silicopn-glass Sandwich gebunden. Die Qualität der mRNA wird mit einem Agilent 2100 Bioanalyser unter Verwendung des RNA 6000 Nano Kit untersucht.

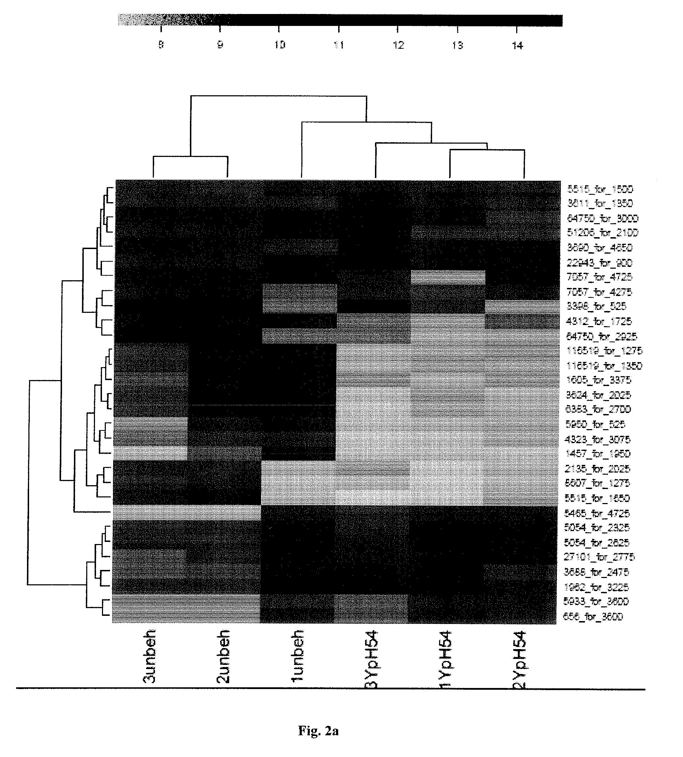

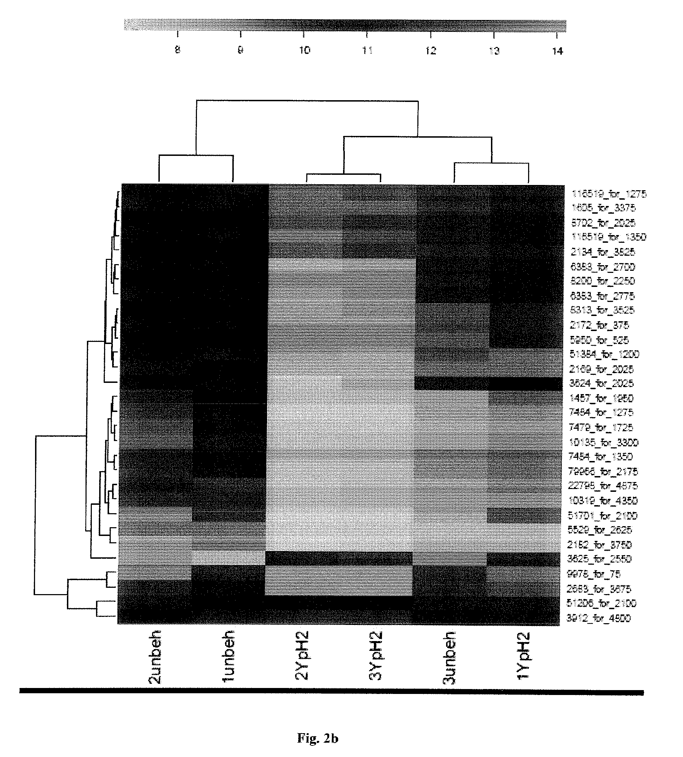

Ein Beispiel für zwei repräsentative Sets mit Chipdaten, wie sie unter Verwendung des beschriebenen Protokolls erhalten wurden, findet sich in **Figs. 2a** und **2b****.** Die Daten dort wurden durch Analyse der Marker von humanen Fibroblasten erhalten, die mit Extrakt aus Yarrowia-Zellen behandelt wurden, die normal bei pH 5,4 kultiviert wurden (**Fig. 2a**) oder Stress bei pH 2 ausgesetzt wurden (**Fig. 2b**).

Die Ergebnisse der Versuche sind in **Tab. 2** zusammengefasst.

**Tabelle 2: Regulation von dermatologisch relevanten Fibroblastenmarkern nach Behandlung mit Extrakten von Saccharomyces cerevisiae-Kulturen, die durch erhöhte Temperatur, Behandlung mit Wasserstoffperoxid oder Exposition gegenüber Medium mit niedrigem pH gestresst wurden.**

| Hefestamm | Stressbedingung | Gesamtzahl der induzierten oder reprimierten Marker | Zahl der stark induzierten oder reprimierten Marker (≥2) |
|---|---|---|---|
| *Saccharomyces cerevisiae* | T | 273 | 1 |
| *Saccharomyces cerevisiae* | pH | 32 | 14 |
| *Saccharomyces cerevisiae* | H₂O₂ | 292 | 3 |

Die unterschiedlichen Expressionsprofile der mit den Extrakten auf verschiedene Weise gestresster Hefen behandelten Zellen zeigen, dass die Hefeextrakte unterschiedliche Eigenschaften aufweisen und es sich somit um unterschiedliche und unterscheidbare Produkte handelt.

### Beispiel 3: Vergleichende Untersuchung der Wirkung von Extrakten von pH-gestressten Zellen verschiedener Hefegattungen auf menschliche Fibroblasten

Es wurde untersucht, inwiefern sich die Zellen verschiedener Hefegattungen eignen, um dermatologisch besonders wirkungsvolle Extrakte herzustellen.

Die Versuchsbedingungen entsprachen den in Beispiel 2 genannten für die Herstellung von Extrakten pHgestresster Zellen, abgesehen davon, dass Extrakte nicht nur von einem Stamm, sondern von *Pichia* CBS1991, *Yarrowia lipolytica* und *Saccharomyces cerevisiae* mit humanen Fibroblasten kontaktiert und ihre Wirkungen verglichen wurden.

**Tabelle 3: Regulation von dermatologisch relevanten Fibroblastenmarkern nach Behandlung mit Extraktenn von Kulturen verschiedener Hefestämme, die durch Exposition gegenüber Medium mit niedrigen pH-Werten gestresst wurden.**

| Hefestamm | pH-Wert | Art der Regulation | Zahl der induzierten oder reprimierten Marker |
|---|---|---|---|
| *Yarrowia lipolytica* | 2 | Induziert | 148 |
| | | Reprimiert | 65 |
| | 5,4 | Induziert | - |
| | | Reprimiert | - |
| *Saccharomyces cerevisia* | 2 | Induziert | 31 |
| | | Reprimiert | 1 |
| | 5,4 | Induziert | - |
| | | Reprimiert | - |
| *Pichia CBS1991* | 3 | Induziert | - |
| | | Reprimiert | - |
| | 5,4 | Induziert | - |
| | | Reprimiert | - |

Die in **Tab. 3** zusammengefassten Ergebnisse zeigen, dass die pH-gestressten Hefezellen der Gattung *Yarrowia* in dem Sinne besonders vorteilhaft auf Haut wirken, dass sie bei der Kontaktierung von humanen Fibroblasten besonders viele Hautmarker positiv regulieren.

### Beispiel 4: Vergleich der Wirkung unterschiedlicher Stressbedingungen auf Hefezellen der Gattung Yarrowia mit Hinblick auf deren dermatologische Wirksamkeit nach Verarbeitung zu Extrakten und Anwendung auf menschliche Fibroblasten.

Nach Identifizierung von Hefezellen der Gattung *Yarrowia* als biotechnologisch und dermatologisch besonders vorteilhafter Quelle von Hefeextrakten für Hautanwendungenwurden verschiedene Stressbedingungen und Kombinationen davon mit den folgenden Ergebnissen verglichen, um ein besonders vorteilhaftes Herstellungsverfahren für derartige Hefeextrakte aufzufinden.

Die Extrakte wurden jeweils unter Verwendung eines Lösungsmittels auf wässriger und auf alkoholischer Basis hergestellt, um die jeweiligen Wirkungen vergleichen zu können.

**Tabelle 4: Regulation von dermatologisch relevanten Fibroblastenmarkern nach Behandlung mit Extraktion von Yarrowia-Kulturen, die unter verschiedenen Stressbedingungen und Kombinationen davon gestresst wurden.**

| Versuch # | Stressbedingung(en) | Wasser (w) oder Propandiol (p)-Aufschluss | Art der Regulation | Zahl der induzierten oder reprimierten Marker |
|---|---|---|---|---|
| 1 | - | w | Induziert | 22 |
| 2 | | w | Reprimiert | 1 |
| 3 | - | p | Induziert | 8 |
| 4 | | p | Reprimiert | - |
| 5 | 37 °C | w | Induziert | 125 |
| 6 | | w | Reprimiert | 119 |
| 7 | 37 °C | p | Induziert | 2 |
| 8 | | p | Reprimiert | 1 |
| 9 | pH 2 bei 37 °C | w | Induziert | 30 |
| 10 | | w | Reprimiert | 6 |
| 11 | pH 2 bei 37 °C | p | Induziert | 1 |
| 12 | | p | Reprimiert | - |
| 13 | pH2 → 37 °C | w | Induziert | 110 |
| 14 | | w | Reprimiert | 93 |
| 15 | pH2 → 37 °C | p | Induziert | 4 |
| 16 | | p | Reprimiert | 1 |
| 17 | 37 °C → pH2 | w | Induziert | **154** |
| 18 | | w | Reprimiert | **118** |
| 19 | 37 °C → pH2 | p | Induziert | 3 |
| 20 | | p | Reprimiert | 1 |

Die in **Tabelle 4** zusammengefassten Ergebnisse zeigen zunächst, dass ein Aufschluss der Zellen unter Verwendung einer wässrigen Lösung gegenüber dem Aufschluss unter Verwendung einer alkoholischen Lösung vorteilhaft ist, da im ersten Fall eine deutlich höhere Zahl an dermatologisch relevanten Fibroblastenmarkern in dermatologisch vorteilhaft wirkender Richtung induziert bzw. reprimiert ist.

Weiterhin zeigt sich, dass die Kombination der Stressfaktoren Exposition gegenüber Medium mit niedrigem pH gefolgt von Temperaturerhöhung die höchste positive Regulation dermatologisch relevanter Marker bewirkt und daher anderen Stressbedingungen gegenüber bzw. deren Kombination vorteilhaft ist.

## Patentansprüche

1. Verfahren zum Herstellen eines dermatologisch wirksamen Hefeextraktes, umfassend folgende Schritte:
a) Bereitstellen einer Vorkultur der Hefezellen,
c) wenigstens fünfzehnminütiges Kultivieren der Zellen bei einem pH-Wert von 1,8 - 4,
d) Ernten der Zellen und
e) Lysieren der Zellen

2. Verfahren nach Anspruch 1, weiter umfassend:
b) wenigstens einstündiges Kultivieren der Zellen bei einer Temperatur von 34 - 39 °C und einem pH-Wert > 5,

3. Verfahren nach einem der Ansprüche 1 oder 2, wobei Schritt e) unter Verwendung eines Lysemittels auf wässriger Basis erfolgt.

4. Verfahren nach einem der Ansprüche 2 bis 3, wobei zunächst Schritt b) und anschließend Schritt c) durchgeführt wird.

5. Verfahren nach einem der Ansprüche 1 bis 4, wobei Schritt c) bei einer Temperatur von 34 - 39 °C durchgeführt wird.

6. Verfahren nach einem der Ansprüche 2 bis 5, wobei Schritt b) 3 - 5 Stunden andauert.

7. Verfahren nach einem der Ansprüche 1 bis 6, wobei Schritt c) 45 - 75 Minuten andauert.

8. Verfahren nach einem der Ansprüche 2 bis 7, wobei die Schritte b) und c) bei einer Temperatur von 36 - 38°C durchgeführt wird.

9. Verfahren nach einem der Ansprüche 1 bis 8, wobei Schritt c) bei einem pH-Wert von 1,9 - 2,2 durchgeführt wird.

10. Verfahren nach einem der Ansprüche 1 bis 9, wobei es sich um eine Hefezelle aus der Gruppe von Gattungen handelt, die Yarrowia, Saccharomyces und Picchia, bevorzugt Yarrowia, umfasst.

11. Verfahren nach einem der Ansprüche 2 bis 10, wobei Schritt b) 3 - 5 Stunden lang bei einer Temperatur von 36 - 38 °C und Schritt c) bei einem pH-Wert von 1,9 - 2,2, bei einer Temperatur von 36 - 38 °C und 45 - 75 Minuten lang durchgeführt wird und es sich bei den verwendeten Hefezellen um Hefezellen der Gattung Yarrowia handelt.

12. Hefeextrakt, erhältlich nach einem der Verfahren nach Anspruch 1 bis 11.

13. Dermatologisch wirksames Mittel umfassend Hefeextrakt aus Hefezellen der Gattung Yarrowia oder Hefeextrakt nach Anspruch 12.

14. Dermatologisch wirksames Mittel nach Anspruch 13, wobei das Mittel haut- und/oder gewebestraffend wirkt.

15. Nahrungsergänzungmittel umfassend Hefeextrakt nach Anspruch 12.

16. Dermatologisch wirksames Mittel nach Anspruch 13 oder 14 oder Nahrungsergänzungsmittel nach Anspruch 15, wobei der Proteinanteil im Hefeextrakt 0,5 - 50 mg/l beträgt.

17. Verfahren zur kosmetischen Behandlung von Haut und/oder Haar, umfassend topische Anwendung des Hefeextraktes nach Anspruch 12 oder einem dermatologisch wirksamen Mittel nach einem der Ansprüche 13, 14 oder 16.

18. Hefeextrakt nach Anspruch 12 zur Herstellung eines Medikamentes.

19. Hefeextrakt nach Anspruch 12 zur Herstellung eines Medikamentes gegen Adipositas, Diabetes, Artherosklerose, entzündlichen Krankheiten oder kardiovaskuläre Krankheiten oder zur Wundbehandlung

20. Verwendung des Hefeextraktes nach Anspruch 12 zur in vitro-Stimulierung der Proliferation von Keratinozyten, Fibroblasten und/oder Adipozyten.
